(19) Europäisches Patentamt
European Patent Office
Office européen des brevets

(11) **EP 2 314 327 B2**

(12) **NEW EUROPEAN PATENT SPECIFICATION**
After opposition procedure

(45) Date of publication and mention
of the opposition decision:
**20.09.2017 Bulletin 2017/38**

(45) Mention of the grant of the patent:
**18.12.2013 Bulletin 2013/51**

(21) Application number: **10013184.6**

(22) Date of filing: **04.05.2004**

(51) Int Cl.:
*A61L 27/20* (2006.01)     *A61L 27/52* (2006.01)
*A61K 47/36* (2006.01)     *A61K 9/00* (2006.01)
*A61K 35/34* (2015.01)

(54) **INJECTABLE CROSS-LINKED POLYMERIC PREPARATIONS AND USES THEREOF**

INJIZIERBARE VERNETZTE POLYMERE ZUSAMMENSETZUNG UND DEREN VERWENDUNG

PRÉPARATIONS POLYMÉRIQUES RÉTICULÉES INJECTABLES ET LEURS UTILISATIONS

(84) Designated Contracting States:
**AT BE BG CH CY CZ DE DK EE ES FI FR GB GR HU IE IT LI LU MC NL PL PT RO SE SI SK TR**

(30) Priority: **05.05.2003 IL 15577403**

(43) Date of publication of application:
**27.04.2011 Bulletin 2011/17**

(62) Document number(s) of the earlier application(s) in accordance with Art. 76 EPC:
**04731094.1 / 1 620 140**

(73) Proprietor: **Ben-Gurion University Of The Negev Research
And Development Authority
84105 Beer Sheva (IL)**

(72) Inventors:
• **Cohen, Smadar**
  **84836 Beer Sheva (IL)**
• **Leor, Jonathan**
  **Chedera 38329 (IL)**

(74) Representative: **Vossius & Partner
Patentanwälte Rechtsanwälte mbB
Siebertstrasse 3
81675 München (DE)**

(56) References cited:
**WO-A-99/15211     US-A- 4 604 394
US-A- 5 709 854     US-A- 6 136 334**

• **OSTBERG T ET AL: "Calcium alginate matrices for oral multiple unit administration: II. Effect of process and formulation factors on matrix properties", INTERNATIONAL JOURNAL OF PHARMACEUTICS, ELSEVIER BV, NL, vol. 97, no. 1-3, 15 August 1993 (1993-08-15), pages 183-193, XP023736446, ISSN: 0378-5173, DOI: DOI:10.1016/0378-5173(93)90138-6 [retrieved on 1993-08-15]**
• **MARTINSEN A ET AL: "ALGINATE AS IMMOBILIZATION MATERIAL: III. DIFFUSIONAL PROPERTIES", BIOTECHNOLOGY AND BIOENGINEERING - COMBINATORIAL CHEMISTRY, WILEY, NEW YORK, NY, US, vol. 39, no. 2, 1 January 1992 (1992-01-01), pages 186-194, XP000991145, DOI: DOI:10.1002/BIT.260390210**

EP 2 314 327 B2

**Description**

**Field of the Invention**

The present invention is defined by the claims. It relates to the use of injectable pharmaceutical preparations containing cross-linked polymer, particularly alginate, as an active ingredient, which polymer forms a hydrogel *in vivo*. The invention relates to the various uses of the injectable cross-linked alginate preparations and to methods of treatment employing the same, particularly repair of cardiac tissue damage and ablation of cardiac arrhythmias.

**Background of the Invention**

All publications mentioned throughout this application are fully incorporated herein by reference, including all references cited therein.

Myocardial infarction (MI) results in acute loss of myocardium and in an abrupt increase in loading conditions that induces left ventricular (LV) remodeling [Sutton, M.G. and Sharpe, N. (2000) Circulation 101:2981-2988; Mann, D.L. (1999) Circulation 100:999-1008; Jugdutt, B.I. (2003) Circulation 108:1395-1403]. The early phase of LV remodeling involves expansion of the infarct zone, which may result in early ventricular rupture or aneurysm formation. Late remodeling involves the entire LV and is associated with time-dependent dilatation, recruitment of border zone myocardium into the scar, distortion of ventricular shape and mural hypertrophy. It results in progressive deterioration in contractile function, heart failure and death [Sutton, M.G. and Sharpe, N. (2000) *id ibid.;* Mann, D.L. (1999) *id ibid.;* Jugdutt, B.I. (2003) *id ibid.*]. Cessation or reversal of progressive chamber remodeling is an important aim of heart failure therapy. However, current clinical interventions to minimize the devastating effects of heart attack are frequently not sufficient to prevent irreversible damage, LV remodeling and subsequent development of heart failure and death [Khand, A.U. et al. (2001) Eur. Heart J. 22:153-164; Jessup, M. and Brozena, S. (2003) N. Engl. J. Med. 348:2007-2018; Redfield, M.M. (2002) N. Engl. J. Med. 347:1442-1444]. However, in the last decade, several research groups including the present inventors have shown that direct injections of cell suspensions of fetal or neonatal cardiac myocytes into experimental myocardial infarcts improved remodeling and function of the heart [Etzion, S. et al. (2001) J Mol Cell Cardiol; 33:1321-1330; Leor, J. et al. (2003) Expert Opin. Biol. Ther. 3:1023-39]. Others replicated those encouraging findings by using skeletal myoblasts bone-marrow-derived cells or embryonic stem cells. Recent reports suggest that endogenous cardiac stem cells may be able to proliferate in the myocardium under certain circumstances, and can migrate from bone marrow to the heart and possibly contribute to repair following cardiac disease [Beltrami, A.P.et al. (2003) Cell; 114:763-776].

The promising results of cardiac cell transplantation in animal models has been partially attributed to reconstruction of the extracellular matrix (ECM), which maintained the structure, thickness, and elasticity of the LV wall [Etzion, S. et al. (2001) J Mol Cell Cardiol; 33:1321-1330]. However, cell transplantation approaches may be of little clinical benefit when the local cardiac structure cannot support cell seeding because it is absent or seriously damaged. The concept of tissue engineering might solve this problem by using 3-D biomaterial scaffolds that replace the missing or damaged infrastructure (ECM) and provides a temporary support for self or implanted cells [Leor, J. et al. Circulation (2000); 102:III56-61].

Previously, the present inventors showed how implantation of cardiomyocyteseeded alginate scaffold onto infarcted myocardium can prevent LV remodeling and dysfunction [Leor, J. *et al.* (2000) *id ibid.*]. This strategy, however, might be limited due to lack of appropriate cells, risk of surgical procedure, general anesthesia and restricted access to LV septum and inferior wall. The aim of the present study was to investigate whether injection of a novel alginate-based biomaterial can efficiently preserve the structure and function of the LV after acute MI while providing biological scaffolding for healing and self-repair. Here, the inventors present evidence for using this approach and suggest that this method has advantages over the strategies commonly utilized.

Biopolymer injection has been used so far to support and promote the engraftment of co-transplantable cells.

Previously, injectable alginate formulations were based on formulations of slowly polymerizing calcium alginate gels [WO 94/25080]. These formulations were used to deliver large number of chondrocytes by means of injection, for the purpose of generating new cartilage. The endoscopic treatment of vesicoureteral reflux was attempted by the injection of alginate-chondrocytes. However, the behavior of the system was less than satisfactory.

Injectable compositions containing cells were produced by suspending suitable cells in medium My99, mixing the cell suspension with an equal part of 2% sodium alginate solution, and then adding solid calcium sulfate powder to initiate cross-linking of the alginate to form a gel. Such compositions typically contain 1% sodium alginate in a hydrogel with insoluble calcium sulfate in an amount of 200 mg per ml of suspension. A small amount of calcium chloride may be carried over into the composition from the cell suspension. Experience indicates that such suspensions have a latent period in the order of one hour, followed by a rapid increase in viscosity to produce a relatively hard, even brittle gel within half an hour of the viscosity increase [WO 94/25080].

However, the consistency of hydrogel-cell suspensions of the type described above is not totally satisfactory for the purpose of injection into patients in need. In some cases, the hydrogel-cell suspension hardens before it can be injected into the patient. In particular, the shortcoming of these injectable cell-alginate formulations which disallows its use in clinical trials is the inconsistent performance between lots, due to poor distribution of the components during formulation.

US Patent No. 6,134,334 describes non-injectable, aqueous pharmaceutical vehicles containing gelling material, which can be used as a drug delivery system, or specifically as corneal protective compositions or ablative corneal shield compositions.

WO 99/15211 describes an injectable composition, having a consistency similar to POLYTEF™ Teflon paste at injection.

US Patent No. 5,709,854 describes injectable solutions of cells and polymers, which gel *in vivo* and are used for promoting tissue formation. Specifically, the injectable alginate solution was polymerized using calcium sulfate. The alginate solution was liquid only for a limited period of time (between 30 and 45 minutes, at 4°C), contained chondrocytes, and was used for cartilage regeneration.

Other injectable polymeric pharmaceutical compositions have been described. For example, US Patent No. 6,129,761 describes slowly polymerizing hydrogels which used for the delivery of cells by injection. Specifically, the publication describes cell-polymer suspensions, wherein the polymer may be, *inter alia,* an alginate, and is intended for improving the implantation of the cells.

US Patent No. 6,171,610 describes the generation of new tissue by liquid hydrogel compositions which comprise a hydrogel and tissue precursor cells.

The techniques described in these references necessarily use cells. This may be a considerable disadvantage. Moreover, the methods of preparing the injectable polymer, for example the method described in WO 94/25080, are different from the method provided in the present invention.

An injectable biopolymer that prevents negative LV remodeling and preserves cardiac function after myocardial infarction was described by Karen L. Christman [International Conference on Engineering Tissue Growth, Pittsburg, Pennsylvania, USA, March 17-20, 2003]. The author stated that injectable fibrin glue may serve as an internal wall support and/or tissue engineering scaffold to prevent deleterious ventricular remodeling and deterioration of cardiac function. While this publication proposes to use cell-free compositions, it is to be noted that *in vivo*, fibrin glue can be retained as such for only about 7 days, and, moreover, it is immuno- and thrombogenic. These are evident disadvantages in the rather long process of tissue regeneration.

WO97/44070 (by the present inventors) describes implantable polysaccharide, e.g. alginate, sponges for use as a matrix, substrate or scaffold for replacement or repair of tissue that has been removed or damaged. The sponges described in this publication are not injectable, and require surgical intervention. Avoiding surgery would be a major advantage.

US 5,776,445 describes an ophthalmic delivery system comprising an alginate which has a particular proportion of guluronic acid, which undergoes a change from dissolved phase to a gel phase upon contacting the lacrimal fluid.

In search for a composition for promoting repair of damaged tissues, the inventors found that injectable polymeric solutions may be useful. More specifically, the present inventors developed a novel hydrogel, a cross-linked alginate, which can be maintained in liquid form indefinitely (under constant conditions) and only gels *in vivo*. Thus, it can serve as an optimal material to be used for tissue repair.

It is therefore an object of the present invention to provide such injectable solution, which contains non-immunogenic, non-enzymatically degradable, bio-erodible polymers, as mentioned in claim 1.

It is a further object of the present invention to provide compositions comprising such injectable solutions in which the said polymer is alginate, particularly cross-linked alginate.

Furthermore, another object of the present invention is the use of said cross-linked alginate in the preparation of injectable solutions for promoting tissue repair and regeneration, particularly for the repair of damaged cardiac tissue.

These and other objects of the invention will become apparent as the description proceeds.

Summary of the Invention

As described herein, the present inventors developed a cross-linked alginate biomaterial which flows as liquid but still maintains sufficient consistency until injection into the desired location in the body, where it forms a solid gel. Most surprisingly, injection of this cross-linked alginate biomaterial promotes regeneration of damaged myocardium and increase of its function, without the need of cell co-transplantation. Thus, the use of these injectable polymeric solutions to treat cardiac infarcts may be an efficient replacement for treatments based on embryonic cell transplantation, in the treatment of myocardial infarct (MI) and chronic heart failure (CHF).

Thus, in a first aspect, the present invention refers to the use, as defined in the claims, of a cross-linked polymer solution, whose elastic response becomes equal to or greater than its viscous response when small deformation oscillatory frequencies are applied and reveals shear thinning behavior in a power-law relationship. Preferred polymers to be used by the invention are hydrogel-forming polymers, like for example polysaccharides. More preferably, said polysaccharide is an alginate.

In one embodiment, the alginate solution is cross-linked with bi- or polyvalent cations (calcium ions or others) while the mixture is homogenized to obtain a homogenous cross-linked alginate biomaterial. As defined herein, the typical cross-linked solution comprises a 0.1-4% (w/v) alginate. Moreover, it is storage stable, i.e., it maintains its solution form and syringeability for longs periods of time. Usually, the cross-linked alginate solution is stable at room or lower than room temperature, for a period of at least 24 hours, for seven days, or even for as long as one year.

In another aspect, the present invention provides a method of preparing a cross-linked alginate solution whose elastic response becomes equal to or greater than its viscous response when small deformation oscillatory frequencies are applied and reveals shear thinning behavior in a power-law relationship, wherein said method comprises the steps of:

(a) dissolving sodium alginate, in water or any other suitable aqueous buffer;
(b) cross-linking the alginate solution obtained in step (a) with a suitable cross-linking agent, by adding an aqueous solution of said agent while stirring intensively until a uniform cross-linked alginate solution is obtained.

Thus, in one specific embodiment of the method of preparing the cross-linked alginate solution of the invention, said cross-linking agent is calcium ions, preferably calcium ions which are provided by a 2% (w/v) calcium gluconate solution.

So, the present invention also provides a cross-linked alginate solution prepared by the method described herein.

The cross-linked alginate solution of the invention may be used for promoting repair and regeneration of damaged cardiac tissue.

In a further aspect, the present invention provides a composition comprising as active agent a cross-linked polymer solution as defined in the invention. Examples of polymers which may be used in the cross-linked polymer solution (the active agent comprised in the composition) are hydrogel-forming polymers, such as polysaccharides. Preferably, the polymer is an alginate.

The injectable preparations prepared by the method of the invention are particularly suitable for the treatment of damaged cardiac tissue, following myocardial infarct, and/or for the treatment of chronic heart diseases. In particularly, the injectable preparations manufactured by the method of the invention are intended for thickening the left ventricular wall following a myocardial event.

In one embodiment of the composition of the invention, said damage is selected from the group consisting of myocardial infarction, ischemic, toxic, inflammatory or mechanical myocardial damage.

In another embodiment, the composition of the invention is for use in the prevention and/ or treatment of conditions resulting from myocardial damage, remodeling and dysfunction, wherein said conditions are selected from the group consisting of left ventricular remodeling, infarct expansion, heart failure and ischemic mitral regurgitation. Alternatively, the composition of the invention may be used in the treatment of focal or re-entrant arrhythmias, and in therapeutic angiogenesis.

The composition of the invention is also for use in guiding stem cell chemotaxis and homing to the damaged myocardium.

In a further embodiment, the composition of the invention further optionally contains additional therapeutic agents, wherein said additional therapeutic agents are selected from the group consisting of antibiotics, growth factors, anti-inflammatory drugs, hormones, anti-apoptotic drugs, growth and stem cell stimulating factors.

In an even further embodiment of the composition of the invention, the composition further comprises cells preferably myoblasts, cardiomyocytes, fibroblasts, endothelial cells, progenitors, stem cells or other suitable cells that may promote cardiac angiogenesis and regeneration.

In a yet further aspect, the present invention provides for the use of the cross-linked polymer solution of the invention in the preparation of a pharmaceutical composition for promoting repair and regeneration of damaged tissue, said cross-linked polymer is an alginate, and said tissue is cardiac tissue, more preferably the left ventricular wall. Said damage may be from various sources, for example myocardial infarction, ischemic, toxic, inflammatory or mechanical myocardial damage.

Therefore, in an even further aspect, the present disclosure provides a method of treatment of damaged tissue, comprising administering a cross-linked polymer solution as defined in the invention to a subject in need. Preferably, said polymer is a cross-linked alginate.

In one embodiment, said tissue to be treated is cardiac tissue, preferably, the left ventricular wall. The cross-linked, alginate solution or composition of the invention should be administered to the damaged myocardium, for ablating the arrhythmogenic substrate.

In one additional aspect, the present invention also provides a method of enhancing the expression of SDF-1, comprising administering the cross-linked alginate solution or the composition as defined in the invention, to a subject in need.

In another additional aspect, the present disclosure provides a method of guiding stem cell chemotaxis, or homing, to the damaged heart, comprising administering the alginate solution the cross-linked alginate solution or the composition as defined in the invention, to a subject in need.

In a further additional aspect, the present disclosure presents a method of inducing neovascularization, comprising administering the cross-linked alginate solution or the composition as defined in the invention, to a subject in need.

In an even further additional aspect, the present disclosure provides a method of inducing therapeutic angiogenesis, comprising administering the cross-linked alginate solution or the composition as defined in the invention, to a subject in need.

Further, the present disclosure provides a method of preventing conditions selected from the group consisting of left ventricular remodeling, infarct expansion, heart failure, ischemic mitral regurgitation, comprising administering the cross-linked alginate solution or the composition as defined in the invention, to a subject in need.

In one more additional aspect, the present invention provides a novel and alternative method of treating focal or re-entrant arrhythmias, comprising administering the cross-linked alginate solution or the composition as defined in the invention, to the ablation site of a subject in need.

As such, in a final additional aspect of the invention, a method of improving myocardial contractility is provided, comprising administering the cross-linked alginate solution or the composition as defined in the invention, to a subject in need.

One more aspect of the present disclosure is a method of inducing cardiac cell proliferation, comprising contacting said cells, *in vivo* or *in vitro,* with the cross-linked alginate solution or the composition of the invention.

Lastly, the present invention provides a kit for repairing damaged tissue, comprising:

(a) a cross-linked polymer solution as defined in the invention, or a composition thereof;
(b) means for administering the polymer solution of (a) into the cardiac site of a patient in need;
(c) manual of instructions of how to use said polymer solution.

In one embodiment of the kit provided by the disclosure, said polymer is preferably a cross-linked alginate.

In another embodiment of the kit provided by the disclosure, said means for administering the cross-linked polymer may be any one of a syringe with a 18-27G needle, any suitable percutaneous cardiac delivery system which includes a cardiac delivery device with a guidewire, including electromechanical mapping or MRI guided catheters, and any percutaneous cardiac device designed to assess the myocardium via the left ventricular cavity, the arterial or venous coronary system.

**Brief Description of the Figures**

**Figure 1A-B:** Effect of calcium ion addition on steady shear viscosity of a 1% (w/v) aqueous LF 5/60 alginate solution (LF 5/60 viscosity = 40 cP).

Fig. 1A: 0.3% (w/v) Calcium ions.
Fig. 1B: 0.4% (w/v) Calcium ions.

Abbreviations: visc., viscosity; S.R., Shear Rate.

**Figure 2:** Effect of calcium ion addition on steady shear viscosity of a 1% (w/v) aqueous LVG alginate solution (LVG viscosity = 127 cP). Abbreviations: visc., viscosity; S.R., Shear Rate.

**Figure 3A-C:** Mechanical spectra of 1% (w/v) LF 5/60 alginate solution and the effect of calcium ion cross-linking.

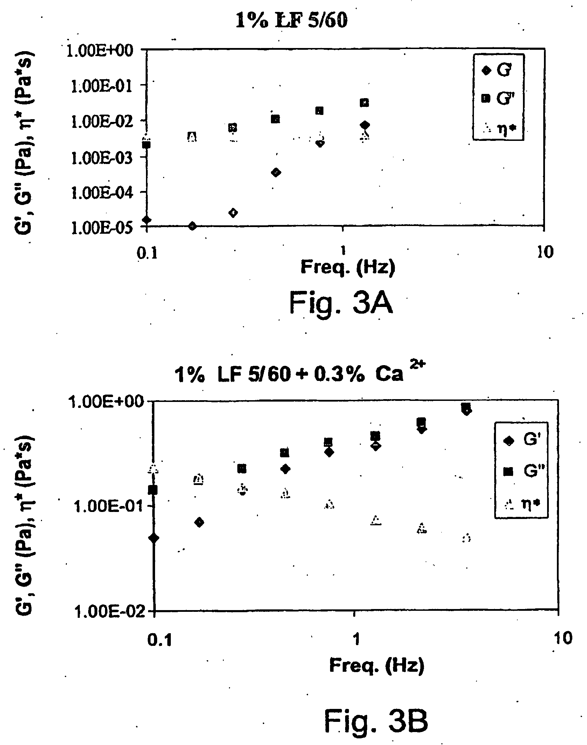

Fig. 3A: No calcium ions.
Fig. 3B: 0.3% (w/v) Calcium ions.

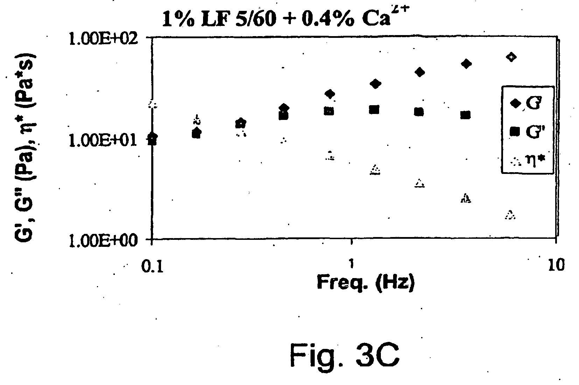

Fig. 3C: 0.4% (w/v) Calcium ions.

Abbreviations: Freq., frequency

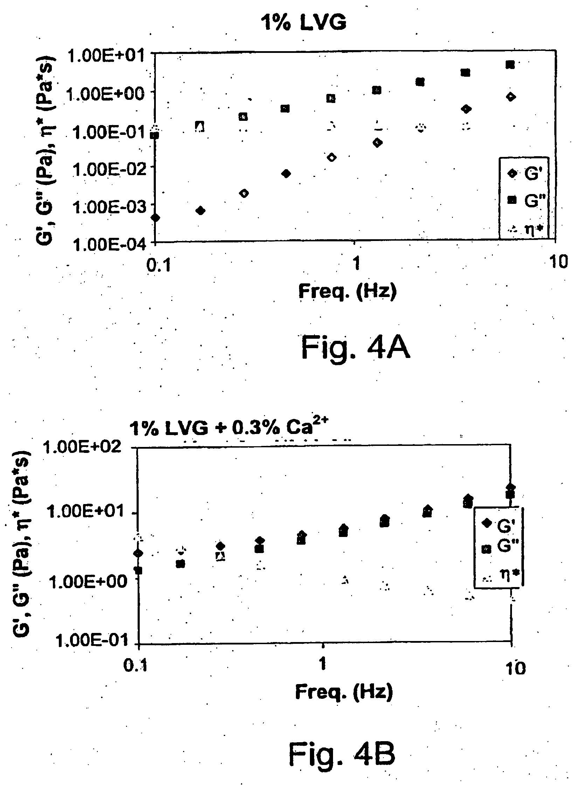

**Figure 4A-B:** Mechanical spectra of 1% (w/v) LVG alginate solution and the effect of calcium ion cross-linking.

Fig. 4A: No calcium ions.
Fig. 4B: 0.3% (w/v) Calcium ions.

Abbreviations: Freq., frequency

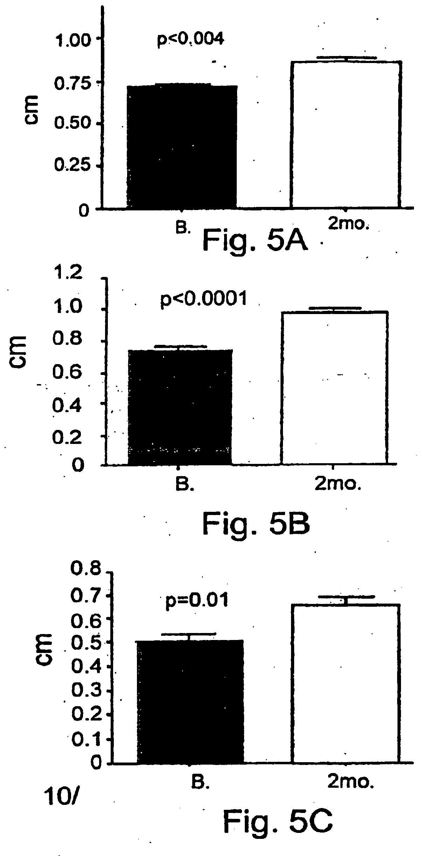

**Figure 5A-H: LV** remodeling by echocardiography.

Fig. 5A: LV diastolic dimension M-mode (alginate).
Fig. 5B: LV diastolic dimension M-mode (control).
Fig. 5C: LV systolic dimension (alginate).

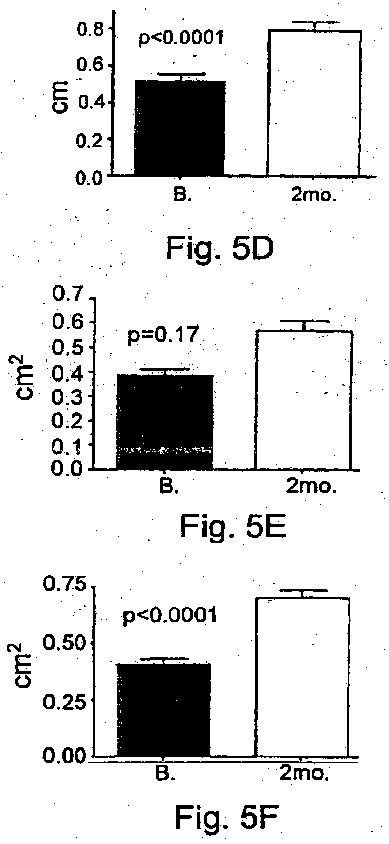

Fig. 5D: LV systolic dimension (control).
Fig. 5E: LV diastolic area (alginate).
Fig. 5F: LV diastolic area (control).

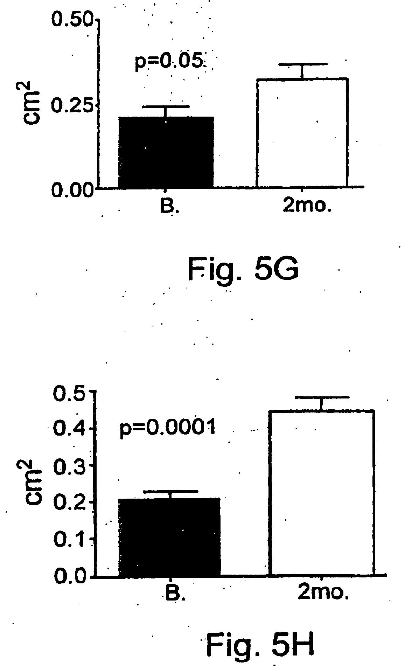

Fig. 5G: LV systolic area (alginate).
Fig. 5H: LV systolic area (control).

Abbreviations: B., baseline; 2 mo., 2 months.

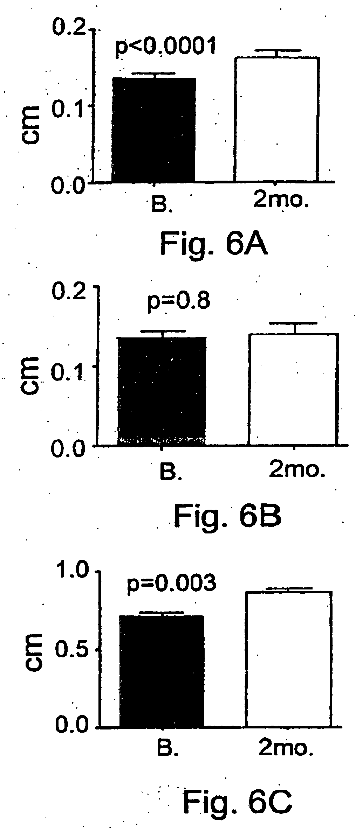

**Figure 6A-F: LV** remodeling by 2-D echocardiography.

Fig. 6A: AW2-D (alginate).
Fig. 6B: AW2-D (control).
Fig. 6C: LV diastolic dimension 2-D (alginate).

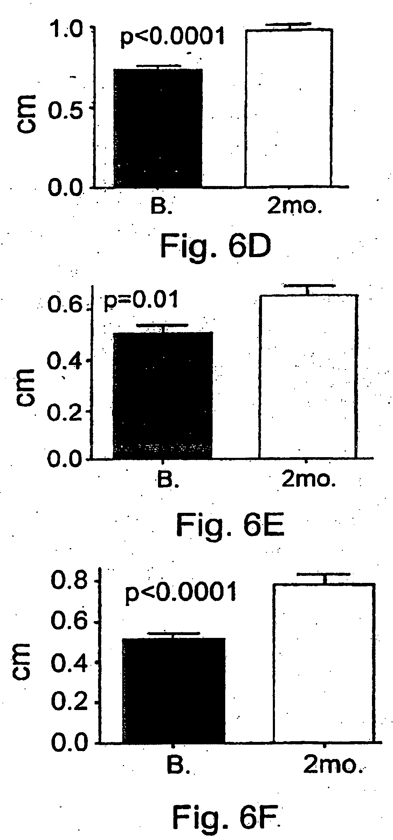

Fig. 6D: LV diastolic dimension 2-D (control).
Fig. 6E: LV systolic dimension 2-D (alginate).
Fig. 6F: LV systolic dimension 2-D (control).

Abbreviations: B., baseline; 2 mo., 2 months.

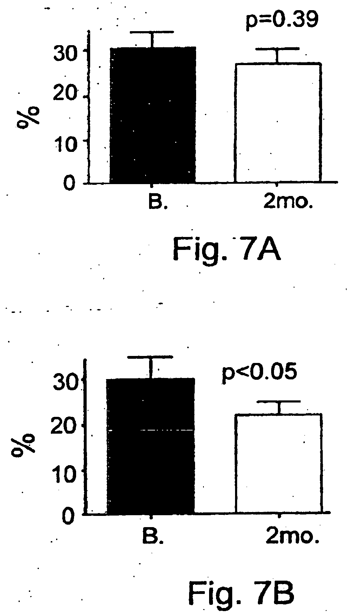

**Figure 7A-D: LV** function by echocardiography.

Fig. 7A: LV fractional shortening (alginate).
Fig. 7B: LV fractional shortening (control).

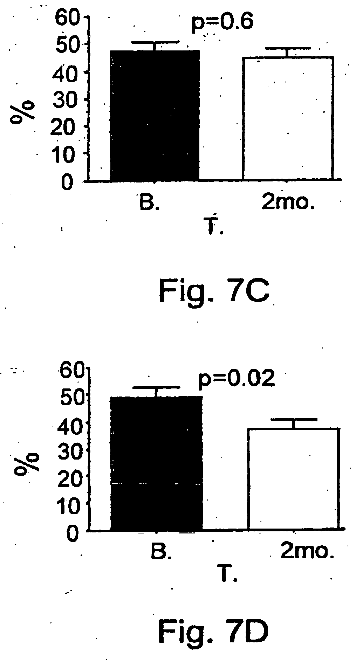

Fig. 7C: LV fractional area change (alginate).
Fig. 7D: LV fractional area change (control).

Abbreviations: B., baseline; 2 mo., 2 months; T., time.

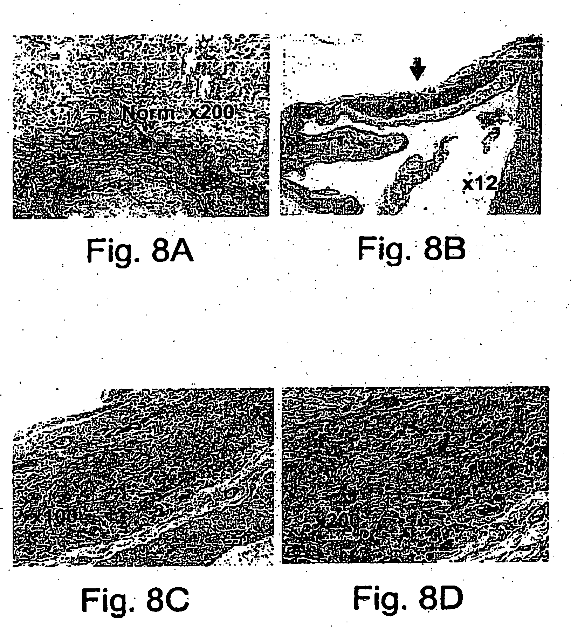

**Figure 8A-D:** Alginate biomaterial induces intensive neoangiogenesis and increases scar thickness

Fig. 8A: Injection of alginate biomaterial into normal myocardium. $\alpha$-SMA antibodies identify neoangiogenesis (arrow) and myofibroblasts.
Fig. 8B: Injection of alginate biomaterial into infarcted myocardium revealed numerous myofibroblasts that populate the scar and increased scar thickness (x12.5 magnification).
Fig. 8C: Higher magnification of Fig. 8B (x100) showing intensive neovascularization (brown staining).
Fig 8D: Higher magnification of Fig. 8B (x200) revealed many myofibroblasts and intensive neovascularization (brown staining).

Abbreviation: Norm., normal.

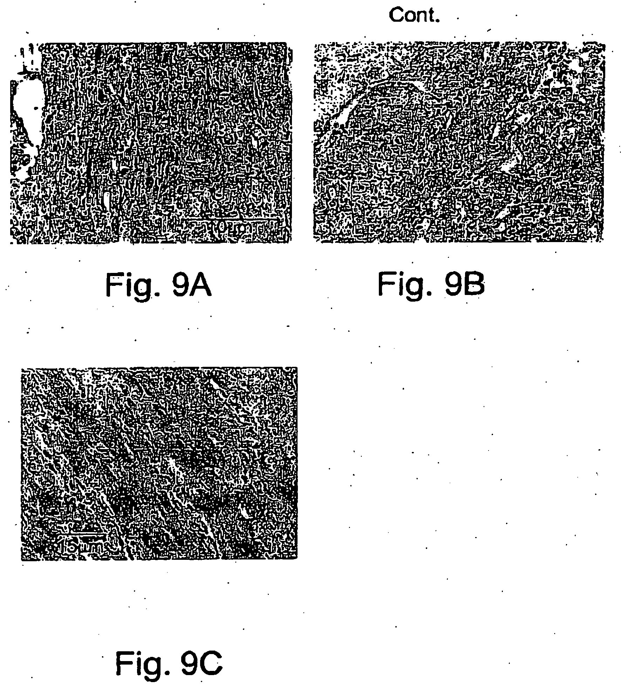

**Figure 9A-C:** Alginate biomaterial injections into infarcted myocardium enhances the expression of SDF-1 - a chemo-attractant for stem cells. Microscopic examination of slides, immunostained with anti-SDF-1 antibody, revealed a robust expression of SDF-1 protein (brown color) at endothelial cells, SMCs, fibroblast and unexpectedly, at cardiomyocytes at the border zone.

Fig. 9A: Anti-SDF-1 staining of samples from cross-linked alginate biomaterial-treated group, x200
Fig. 9B: Anti-SDF-1 staining of samples from control (treated with culture medium), *x*200.
Fig. 9C: Anti-SDF-1 staining of slides from alginate biomaterial treated group, focusing on stained myocytes at the border zone between infarct and normal myocardium.

Abbreviation: Cont., control.

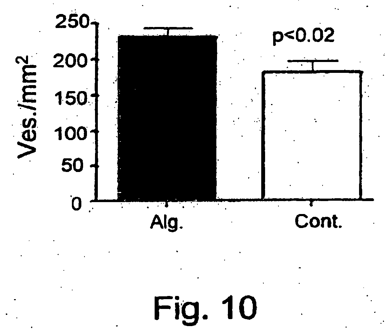

Figure 10: Quantification of angiogenesis (in number of vessels per area). Abbreviations: Ves., vessels; Alg., alginate; cont., control.

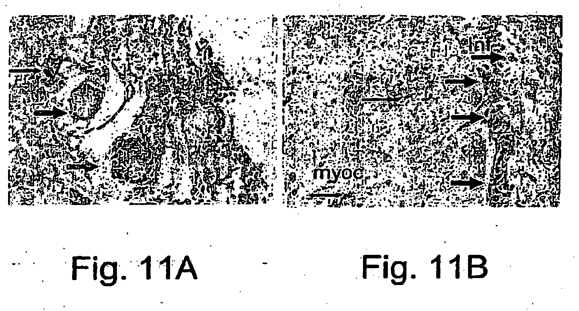

Figure 11A-B: Alginate biomaterial injection induces cell replication and regeneration in infarcted myocardium. Immunostaining with anti Ki67 antibodies at week 8 after LAD occlusion.

Fig. 11A: The pig receiving alginate injection demonstrated high frequency of endothelial cells (arrows) with DNA activity.
Fig. 11B: Examination of the border zone revealed several myocytes (fine arrows), endothelial cells and fibroblasts with positive Ki67 staining.

In contrast, in animals receiving saline (data not shown) there was a high frequency of cells with fibroblast morphology and reactivity with Ki67 within the infarct zone only.
Abbreviations: myoc., myocardium; Inf., infarct.

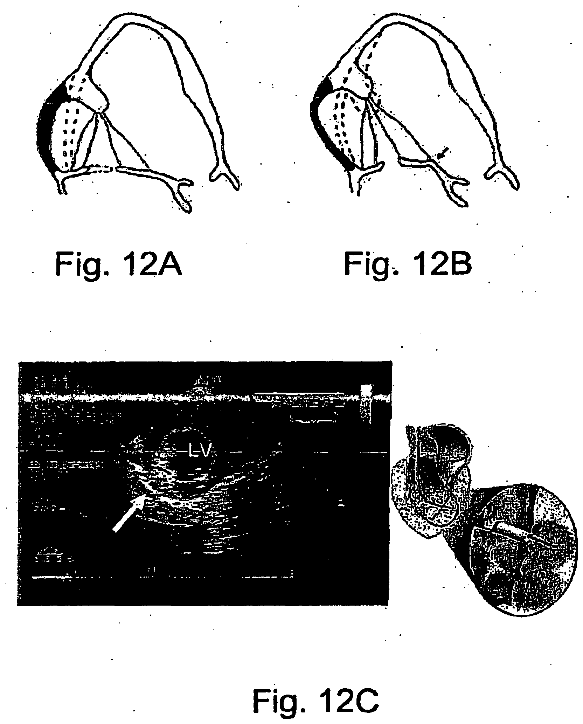

Figure 12A-D: Injectable Tissue Engineering reverses Ischemic Mitral Regurgitation.

Fig. 12A: Schematic of ischemic mitral regurgitation with mitral annulus dilatation.
Fig. 12B: Schematic of ischemic mitral regurgitation with change in the global geometry of the left ventricle and tethering of the mitral leaflet.
Fig. 12C: Injection of alginate based-biomaterial into the infarcted LV postero-lateral segment (arrow) repositions the displaced papillary muscle toward the anterior annulus to relieve tethering and MR.
Fig. 12D: Schematic of alginate injection.

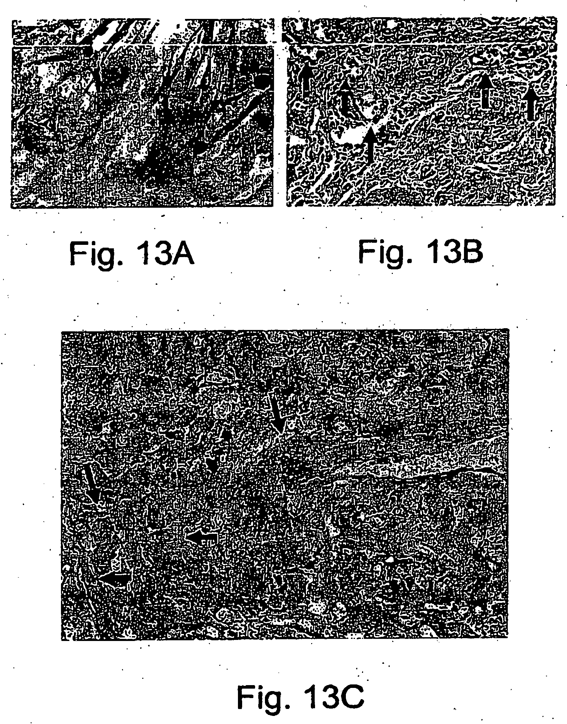

Figure 13A-C: Alginate-myoblast suspension injections showing that the polymer increases cell retention at injection site and enhances angiogenesis.

Fig. 13A: Immunostainig with anti-desmin antibodies identified multinucleated myoblasts (brown staining, x400).

Fig. 13B: H&E stained section of heart treated with injection of myoblast suspension with alginate solution revealed neoangiogenesis and functional vessels filled with red blood cells (x400).

Fig. 13C: Myoblast survival and location within myocardium after 4 weeks. Immunostainig with anti-skeletal, fast myosin heavy chain antibodies revealed skeletal striated myocytes (arrows) at the site of injection (x400).

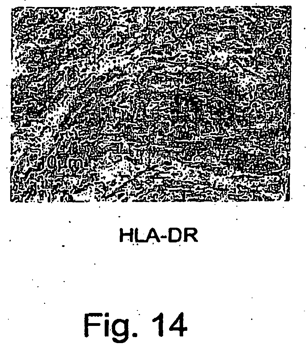

Figure 14: Alginate biomaterial injections enhance stem cell homing to infarcted myocardium.
HLA-DR immunostaining for human progenitors (brown color) revealed that the infused CD133+ progenitor cells homed and colonized the site wherein cross-linked alginate biomaterial was injected, at the scar tissue (one week after transfusion).

## Detailed Description of the Invention

The following abbreviations are used in the present application:

BMP:        bone morphogenic protein
bFGF:       basic fibroblast growth factor
CHF:        chronic heart failure
2-D:        two dimensional
DDW:        double distilled water
ECM:        extracellular matrix
EDA:        end diastolic area
ESA:        end systolic area
GPC:        Gel Permeation Chromatography
IGF:        insulin-like growth factor

| LA: | left atrium |
|---|---|
| LAD: | left anterior descending |
| LV: | left ventricular |
| LVEF: | LV ejection fraction |
| LVID: | LV internal dimension |
| MALLS: | multi-angle laser light scattering |
| MHC: | myosin heavy chain |
| MI: | myocardial infarct |
| Mn: | Molecular number |
| MR: | mitral regurgitation |
| Mw: | Molecular weight |
| PD: | polydispersity |
| PM: | papillary muscles |
| SDF: | stromal cell derived factor |
| SMA: | smooth muscle actin |
| TGF: | transforming growth factor |
| VEGF: | vascular endothelial growth factor |
| WMSI: | wall motion score index |

The promising results of cardiac cell transplantation or tissue engineering in animal models have been partially attributed to reconstruction of the extracellular matrix (ECM), which maintains the structure, thickness, and elasticity of the LV wall. The inventors have investigated whether manipulation of ECM by injections of cross-linked alginate-based biomaterial can efficiently preserve the structure and function of the LV while providing a scaffold for healing and self-repair.

Surprisingly, the inventors found that, as shown in the following Examples, attenuation of LV dilatation and myocardial dysfunction following myocardial infarct by injection of a solution of cross-linked alginate, injected to infarcted myocardium in a rat model, was comparable to that achieved by embryonic cardiomyocytes transplantation.

Thus, the use of injectable polymeric solutions to treat cardiac infarcts may be an efficient replacement for the use of the difficult to obtain embryonic cells, in the treatment of myocardial infarct (MI) and chronic heart failure (CHF).

For this purpose, the present inventors developed a cross-linked alginate biomaterial which flows as liquid but still maintains sufficient consistency until injection into the desired location in the body, where it forms a solid gel. The cross-linked alginate can be kept indefinitely in its flowable form outside the body, at temperatures varying between 0°C and 30ºC, preferably at room temperature (which is between 21°C and 25°C), and only at injection site it forms a solid or semi-solid gel matrix.

Based on its rheological behavior (as detailed below), the cross-linked alginate biomaterial developed herein is defined as entanglement networks, which are distinguished from the strong covalent gels in that they do not have permanent cross-links, are strongly frequency-dependent, have G'-G" crossover, and flow as a liquid at low frequencies (as shown in Example 1).

Thus, in a first aspect, the present invention refers to a cross-linked polymer solution, whose elastic response becomes equal to or greater than its viscous response when small deformation oscillatory frequencies are applied and reveals shear thinning behavior in a power-law relationship.

A shear thinning behavior is characteristic of a solution in which at lower shear rates, the solution is more viscous than a Newtonian Fluid, and at higher shear rates it is less viscous. A solution that exhibits a power-law relationship is one whose viscosity decreases as its shear rate increases.

To achieve a solution with such properties, the cross-linked polymer solution of the invention is prepared from a polymer precursor solution which exhibits Newtonian behavior and whose viscous response is greater than its elastic response when small deformation oscillatory frequencies are applied. A polymer solution that exhibits a Newtonian behavior is also referred to as a Newtonian fluid, i.e., a fluid that has a constant viscosity at all shear rates at a constant temperature and pressure, and in which the rate of deformation is directly proportional to the stress applied to the fluid. Upon cross-linking, the solution's elastic response becomes equal or greater than its viscous response when small deformation oscillatory frequencies are applied.

Said applied small deformation oscillatory frequencies are within the viscoelastic limit of 0.01-100 Hz, which is preferably within the range from about 0.1-10 Hz.

Preferred polymers to be used by the invention are hydrogel-forming polymers, like for example polysaccharides.

More preferably, said polysaccharide is an alginate. An alginate is a watersoluble polysaccharide which, when cross-linked with bivalent cations such as calcium ions, undergoes an increase in viscosity until forming a solid or semi-solid gel.

As illustrated in Example 1, said alginate may be of various Mw, preferably in the range between 10K to 300K Dalton, more preferably between 25K and 250K Dalton.

Cross-linking of the polymer solution of the invention may be via any one of covalent, ionic and hydrogen bonds, in order to create a structured network which entraps water molecules.

In one embodiment, the alginate solution is cross-linked with bi- or polyvalent cations (calcium ions or others) while the mixture is homogenized to obtain a homogenous cross-linked alginate biomaterial.

As defined herein, the typical cross-linked solution comprises a 0.1-4% (w/v) alginate, preferably 0.5-2% alginate. Moreover, it is storage stable, i.e., it maintains its solution form and syringeability for long periods of time. Usually, the cross-linked alginate solution is stable at room or lower than room temperature, for a period of at least 24 hours, preferably at least seven days, more preferably up to one year.

The term syringeability, as used herein, is to be taken to mean that the solution maintains its fluidic properties and can be administered by injection (with a syringe and a needle), catheterization (through catheters or shunts), or any suitable percutaneous cardiac delivery system which includes a cardiac delivery device with a guidewire, including electromechanical mapping or MRI guided catheters, as well as any percutaneous cardiac device designed to assess the myocardium via the left ventricular cavity, the arterial or venous coronary system, and any further suitable method and means for administration of a fluid into any part of the body of a subject in need, particularly non-surgical methods. Thus, the cross-linked alginate solution of the invention may be administered to a subject in need by any one of the means detailed herein above.

The cross-linked alginate biomaterial is flowable and can be injected to body tissues (e.g. infarcted myocardium) via an 18-27G needle. Alternatively, other suitable cardiac delivery system, as described above may be used. At the injection site (*in vivo*), the cross-linked biomaterial forms a gel and becomes solid or semi-solid.

It is important to note that, as referred to herein, a gel consists of a network interspersed with a liquid, usually water in the case of hydrogel. Two common criteria defining a gel are that: (i) it must contain a liquid, and (ii) it must have a network that spans the whole sample.

In another aspect, the present invention provides a method of preparing a cross-linked alginate solution whose elastic response becomes equal to or greater than its viscous response when small deformation oscillatory frequencies are applied and reveals shear thinning behavior in a power-law relationship, wherein said method comprises the steps of:

(a) dissolving sodium alginate in water or any other suitable aqueous buffer;
(b) cross-linking the alginate solution obtained in step (a) with a suitable cross-linking agent, by adding a suitable volume of an aqueous solution of said agent while stirring intensively until a uniform cross-linked alginate solution is obtained.

Cross-linking may be achieved by using ions, altering the pH or changing the temperature. Ionic cross-linkers include metal cations, such as calcium, copper, aluminum, magnesium, strontium, barium, tin, zinc, chromium, di-, tri- and tetrafunctional organic cations. Polyions may be used such as poly(amino acids), poly(ethyleneimine), poly(vinylamine), poly(allylamine), and cationic polysaccharides.

Thus, in one specific embodiment of the method of preparing the cross-linked alginate solution of the invention, said cross-linking agent is calcium ions, preferably calcium ions which are provided by a 2% (w/v) calcium gluconate solution.

It is to be noted that the degree of cross-linking will determine the rate of erosion of the solid depot formed *in vivo* upon injection. The concentration of the polymer in the injectable solution depends on its molecular weight and on the intended degree of cross-linking. The design of the preparation should take into account the factors necessary to achieve an adequate liquid/solid phase transition such as polymer Mw and concentration, and the concentration of the cross linker. Such design is within the capabilities of a person skilled in the art of pharmacy. Example 1 is an illustration of preferred conditions to achieve the polymer solution of the invention.

The cross linked polymer to be used by the invention should be a biocompatible polymer, which is capable of gelling *in vivo* and thus forming a hydrogel depot at the site of injections. The polymer should be non-enzymatically degradable, and bio-erodible. Most importantly, the polymer is preferably non-immunogenic. As shown in the following Examples, the cross-linked alginate solution of the invention is non-immunogenic, since none of the experimental animals injected with the solution displayed any signs of immune reaction towards the cross-linked alginate.

Thus, in essence, the present invention provides a method of preparing a cross-linked alginate solution whose elastic response becomes equal to or greater than its viscous response when small deformation oscillatory frequencies are applied and reveals shear thinning behavior in a power-law relationship, wherein said method comprises the steps of:

(a) dissolving sodium alginate in water or in any one suitable aqueous buffer;
(b) cross-linking the alginate solution obtained in step (a) with calcium ions, by adding a suitable volume of a 2% (w/v) calcium gluconate solution while stirring intensively, until a uniform solution is obtained.

Optionally, the alginate solution obtained in step (a) may be filtered through a series of suitable membrane filters, as for example nylon filters of 1.2, 0.45 and 0.2 $\mu$m, and then proceed to step (b).

So, the present invention also provides a cross-linked alginate solution prepared by the method described herein.

Unexpectedly, the inventors found that the injection of the cross-linked alginate biomaterial by itself could promote regeneration of damaged myocardium and increase of its function without the need for cell co-transplantation.

Consequently, the cross-linked alginate solution of the invention may be used for promoting repair and regeneration of damaged cardiac tissue.

In a further aspect, the present invention provides a composition comprising as active agent a polymer solution as defined in the invention.

Examples of polymers which may be used in the polymer solution (the active agent comprised in the composition) are hydrogel-forming polymers, such as polysaccharides. Preferably, the polymer is an alginate.

The injectable preparations prepared by the method of the invention are particularly suitable for the treatment of damaged cardiac tissue, following myocardial infarct, and/or for the treatment of chronic heart diseases. In particularly, the injectable preparations manufactured by the method of the invention are intended for thickening the left ventricular wall following a myocardial event.

Consequently, the composition of the invention may be used for promoting repair and regeneration of damaged tissue, preferably cardiac tissue, specially the left ventricular wall.

In one embodiment of the composition of the invention, said damage is selected from the group consisting of myocardial infarction, ischemic, toxic, inflammatory or mechanical myocardial damage.

In another embodiment, the composition of the invention is for use in the prevention and/ or treatment of conditions resulting from myocardial damage, remodeling and dysfunction, wherein said conditions are selected from the group consisting of left ventricular remodeling, infarct expansion, heart failure and ischemic mitral regurgitation.

Additionally, it is expected that the injectable preparations of the invention may be suitable for the treatment of tissue damage resulting from arrhythmia. Using electrochemical mapping, the injectable preparation of the invention can be used for "bio-ablation", i.e. ablation of cardiac arrhythmias by injection into arrhythmic foci and pathways.

Cardiac adaptation through hypertrophy may predispose the patient to heart failure and potentially fatal arrhythmias. The mechanism of the arrhythmias is described as either focal or re-entrant. Re-entry is a simple concept, and is the mechanism of most clinically important arrhythmias. It describes the progression of a wave front of electrical activation through cardiac muscle over a pathway that leads back to its point of origin. This completes one cycle of a re-entrant circuit, and providing that certain critical conditions exist, conduction will continue around the circuit again and again to produce a regular arrhythmia.

In any condition in which there is a structural variant providing a similar circular conduction pathway - be it congenital (such as accessory pathway mediated tachycardia, atrioventricular nodal re-entrant tachycardia, and possibly atrial flutter), or acquired (such as ventricular tachycardia after myocardial infarction) - there is the potential for re-entrant arrhythmias. By contrast, when there is severe, generalized disruption of the electrical properties of the myocardium, as occurs in many forms of structural heart disease, re-entrant wave fronts can meander aimlessly through the myocardium without following a fixed path and lead to fibrillation. The mechanism underlying focal arrhythmias is abnormal, rapid, spontaneous electrical activity of a group of cells spreading to the rest of the myocardium.

The aim of catheter ablation via alginate biomaterial injection is to eliminate the arrhythmia by locating and ablating the safest and most accessible point that will either transect and interrupt a re-entrant circuit or eliminate a focus. The technique involves the percutaneous introduction of electrode catheters (insulated wires with electrodes at their tip, much like temporary pacing wires) into the heart under fluoroscopic guidance, in order to record electrical signals from relevant parts of the heart. Once the mechanism of the arrhythmia is established, one of the electrode catheters is navigated to a critical site at which ablative energy (radiofrequency current, which is predictable, effective, and well tolerated) is delivered to create a localized scar that will disrupt the cause of the arrhythmia. In sum, the cross-linked alginate is injected into the re-entry pathway or arrhythmia focus, producing fibrosis and interruption of the arrhythmia circuit.

Thus, alternatively, the composition of the invention may be used in the treatment of focal or re-entrant arrhythmias.

Another use of the composition of the invention is in therapeutic angiogenesis.

The composition of the invention is also for use in guiding stem cell chemotaxis and homing to the damaged myocardium.

In a further embodiment, the composition of the invention further optionally contains additional therapeutic agents, wherein said additional therapeutic agents are selected from the group consisting of antibiotics, growth factors, anti-inflammatory drugs, hormones, anti-apoptotic drugs, growth and stem cell stimulating factors.

Various growth factors can be used as additional therapeutic agents, for example angiogenesis stimulating factors and revascularization enhancing factors, e.g. basic fibroblast growth factor, (bFGF), vascular endothelial growth factor (VEGF), insulin-like growth factor (IGF), members of the TGF-family, bone morphogenic proteins (BMP), platelet-derived growth factors, angiopoietins, and other factors such as myogenic factors, transcription factors, cytokines, and homeobox gene products.

Cytokines, growth factors, and angiogenic factors can be encapsulated in biodegradable microparticles or nanoparticles and embedded in biomaterials, like the cross-linked alginate solution of the invention, to enhance tissue regeneration. Scaffoldings capable of mimicking cellular matrices, as the one generated by the injection of the cross-linked alginate solution of the invention, which gels *in vivo,* upon injection, and have the potential to stimulate the growth of new myocardium as well as direct revascularization, as shown in the following Examples.

In an even further embodiment of the composition of the invention, the composition further comprises cells, preferably myoblasts, cardiomyocytes, fibroblasts, endothelial cells, progenitors, stem cells or other suitable cells that may promote cardiac angiogenesis and regeneration.

As shown in Example 6, injection of myoblasts together with the cross-linked alginate solution of the invention enhanced the retention of the transplanted myoblasts at injection site, induced angiogenesis and the formation of functional vessels. The myoblasts differentiated into multinucleated fibers revealing skeletal striation.

The preparation of pharmaceutical compositions is well known in the art and has been described in many articles and textbooks, see e.g., Remington's Pharmaceutical Sciences, Gennaro A. R. ed., Mack Publishing Co., Easton, PA, 1990, and especially pp. 1521-1712 therein.

In a yet further aspect, the present invention provides for the use of the polymer solution of the invention in the preparation of a pharmaceutical composition for promoting repair and regeneration of damaged tissue. Preferably said polymer is a cross-linked alginate, and said tissue is cardiac tissue, more preferably the left ventricular wall. Said damage may be from various sources, for example myocardial infarction, ischemic, toxic, inflammatory or mechanical myocardial damage.

Therefore, in an even further aspect,the present invention provides a method of treatment of damaged tissue, comprising administering a polymer solution as defined in the invention to a subject in need. Preferably, said polymer is a cross-linked alginate.

In one embodiment, said tissue to be treated is cardiac tissue, preferably, the left ventricular wall. The cross-linked alginate or composition of the invention should be administered to the damaged myocardium, for ablating the arrhythmogenic substrate.

In the present invention, the inventors have shown, for the first time, that injection of alginate-based biomaterial into the infarcted myocardium in a rat model of extensive MI attenuates LV dilatation and myocardial dysfunction (Example 2). The results provide a proof of concept and a novel option of injectable biomaterial scaffolding to preserve LV geometry and function, after severe myocardial damage and to prevent LV dysfunction. This work suggests a viable alternative to the difficulties in achieving appropriate number of functional donor cells for cardiac tissue engineering [Etzion, S. et al. (2001) Am J Cardiovasc Drugs; 1:233-244]. Furthermore, it can be used together with cell delivery to improve cell retention, colonization and survival, by inducing neovascularization and expression of the SDF-1 survival factor.

The mechanism of how the injection of alginate biomaterial results in tissue repair and regeneration is not clear. Possibly, the injection of the cross-linked alginate solution of the invention reduces wall stress by stabilizing chamber size and increasing scar thickness. This mechanism could inhibit infarct expansion and prevent aneurysm formation in this myocardial region. Alternatively, the favorable effects of alginate injection may be beyond its constraint mechanical properties and might be partially related to the induction of neovascularization and regeneration.

Enlargement and spherical deformation of the LV with a concomitant increase in wall stress are the key elements in the pathogenesis of LV remodeling [Sutton and Sharpe (2000) *id ibid;* Mann (1999) *id ibid*.]. This is the rationale for passive external containment by mesh graft implantation, which aims at counteracting progressive left ventricular dilatation and deformation. Several studies using animal models or patients with MI or dilated cardiomyopathy have demonstrated beneficial effects of passive cardiac containment on myocardial structure and function [Kelley, S:T. et al. Circulation 1999; 99:135-142; Pilla, J.J. et al. (2002) Circulation; 106:1207-211; Saavedra, W.F. et al. (2002) J Am Coll Cardiol; 39:2069-2076; Lembcke, A. et al. (2004) Eur J Cardiothorac Surg; 25:84-90]. In addition to constraining cardiac enlargement, the girdling effect is assumed to reduce regional ventricular wall stress and myocardial work. It is possible that the injected biomaterial replaces the injured ECM and provides temporary scaffolding until migrating or implanted cells produce their own ECM and create a stronger scar.

An exciting new finding in the present study is the strong SDF-1 expression in the infarct and border zone of hearts treated with alginate injection. SDF-1 is a key regulator of stem cell homing and chemotaxis. This effect suggests that the injected biomaterial activates a signaling system that attracts stem cells to the injected site, which participate in neovascularization and myocardial regeneration.

Thus, in one additional aspect, the present invention also provides a method of enhancing the expression of SDF-1, comprising administering the cross-linked alginate solution or the composition as defined in the invention, to a subject in need.

Apparently, circulating stem cells home to loci of high SDF-1 concentration, as clearly demonstrated by *in vitro* experiments showing transmigration capacity towards SDF-1 gradients [Kollet, O. et al. Blood 2001; 97:3283-3291;

Lapidot, T. and Petit, I. (2002) Exp Hematol; 30:973-981]. Injection of human SDF-1 into the spleen and bone marrow of immunodeficient mice led to rapid homing of transplanted human stem cells into the spleen and bone marrow [Kollet *et al.* (2001) *id ibid.*]. Other data accord with the SDF-1 concept that stem-cell homing is not only restricted to the bone marrow but can also be found in the wounded heart [Askari, A.T. et al. Lancet 2003; 362:697-703; Pillarisetti, K. and Gupta, S.K. Inflammation 2001; 25:293-300]. Therefore, SDF-1 may have an important role in triggering these homing effects. SDF-1, through CXCR4, induces angiogenesis in vivo. [Salcedo, R. et al. Am J Pathol 1999; 154:1125-1135; Tachibana, K. et al. Nature 1998; 393:591-594; Salvucci, O. et al. Blood 2002; 99:2703-2711]. SDF-1 and CXCR4 directly enhance cell survival [Broxmeyer, H.E. et al. (2003) J. Leukoc. Biol. 73:630-638; Yamaguchi J-I et al. (2003) Circulation 107:1322-1328]. Yamaguchi and colleagues [Yamaguchi *et al.* (2003) *id ibid.*] showed that the effect of SDF-1 on neovascularization appears to result from its ability to enhance the recruitment and incorporation of transplanted endothelial progenitor cells (EPCs). In another study, Askari and colleagues showed that transplantation of genetically modified cardiac fibroblasts expressing SDF-1 combined with stem-cell mobilization by granulocyte colony-stimulating factor can restore depressed myocardial function [Askari *et al.* (2003) *id ibid.*]. Furthermore, SDF-1 directly enhances survival/anti-apoptosis of progenitor cells through CXCR4 [Broxmeyer, H.E. *et al.* (2003) *id ibid.; Yamaguchi J-I et al.* (2003) *id ibid.*].

Thus, in another additional aspect, the present invention provides a method of guiding stem cell chemotaxis, or homing, to the damaged heart, comprising administering the cross-linked alginate solution the cross-linked alginate solution or the composition as defined in the invention, to a subject in need.

An integral component of the remodeling process is the development of neoangiogenesis within the myocardial infarct scar. Under normal circumstances, the contribution of neoangiogenesis to the infarct-bed capillary network is insufficient to keep pace with the tissue growth required for contractile compensation, and is unable to support the greater demands of the hypertrophied but viable myocardium. The relative lack of oxygen and nutrients to the hypertrophied cardiomyocytes might be an important etiological factor in the death of otherwise viable myocardium, resulting in progressive infarct extension and fibrous replacement. Because late reperfusion of the infarct vascular bed in both humans and animal models significantly benefits ventricular remodeling, neoangiogenesis might improve cardiac function by preventing loss of hypertrophied but otherwise viable cardiac myocytes.

Microscopic examination of the treated hearts (Fig. 8A-D) revealed that alginate injection recruited many myofibroblasts and promoted intensive angiogenesis in the infarcted myocardium. It is likely that restoration of the blood flow toward the peri-infarct region may be associated with an overall better infarct healing [Kocher, A.A. et al. (2001) Nat Med; 7:430-436]. The beneficial role of the neoangiogenesis scaffolding as an 'erectile force' provided by a blood-filled coronary vascular bed has been originally suggested by Salisbury *et al.* [Salisbury, P.F. et al. (1960) Circ Res; 8: 794-800] and further supported by results of studies of Braunwald [Braunwald, E. Circulation 1989; 79:441-444]. Moreover, Hale and Kloner reported thicker scars (showing an increased resistance to radial stress) in the infarcts of late-reperfused rats [Hale, S.L. and Kloner, R.A. Am Heart J 1988; 116:1508-1513].

Taken together, neovascularization after extensive MI might prevent cell death, maintain viability and prevent LV remodeling and dysfunction [Kocher *et al.* (2001) *id ibid.*; Abbate, A. et al. J Cell Physiol 2002; 193:145-153; Abbate, A. et al. Circulation 2002; 106:1051-1054] It might provide biological scaffolding and passive constraint that prevent LV dilatation and dysfunction.

In view of the results discussed above, in a further additional aspect, the present invention presents a method of inducing neovascularization, comprising administering the cross-linked alginate solution or the composition as defined in the invention, to a subject in need.

Efforts to reduce the extent of muscle damage from acute MI have been successful in many ways, but the problem of post-infarction scarring, remodeling, dilatation, and consequent heart failure remains. Unfortunately, the benefits of contemporary infarct-limiting strategies, such as early reperfusion, sophisticated fibrinolytic and anti-platelet treatments, and myocardial protection, are all approaching their apparent limits. Consequently, researchers are aggressively developing new strategies aimed at replacing old infarcted myocardium with new tissue, rather than focusing exclusively on limiting or preventing the original damage. Therapeutic angiogenesis has emerged as a potentially novel approach to treating symptomatic ischemic heart disease not amenable to conventional percutaneous or surgical approaches. Such "no option" patients may represent as many as 12% of all those referred for treatment of occlusive coronary disease. By inducing new vessel formation and collateral circulation, therapeutic angiogenesis enhances ischemic tissue perfusion and viability, healing and prevents progressive myocardial damage.

Thus, in an even further additional aspect, the present invention provides a method of inducing therapeutic angiogenesis, comprising administering the cross-linked alginate solution or the composition as defined in the invention, to a subject in need.

The terms neo-vascularization and angiogenesis are used herein interchangeably.

Further, the present disclosure provides a method of preventing conditions selected from the group consisting of left ventricular remodeling, infarct expansion, heart failure, ischemic mitral regurgitation, comprising administering the cross-linked alginate solution or the composition as defined in the invention, to a subject in need.

In one more additional aspect, the present disclosure provides a novel and alternative method of treating focal or re-entrant arrhythmias, comprising administering the cross-linked alginate solution or the composition as defined in the invention, to the ablation site of a subject in need.

The cross-linked alginate of the present invention may be used for improving systolic function, which is defined by the characteristics of heart muscle contraction, such as force, maximal pressure, power, ejection time, cardiac output and etc.

As such, in a final additional aspect of the disclosure, a method of improving myocardial contractility is provided, comprising, administering the cross-linked alginate solution or the composition as defined in the invention, to a subject in need.

Interestingly, the present inventors have demonstrated that upon injection of the cross-linked alginate solution of the invention, cardiomyocytes were induced to proliferate. This was illustrated in Example 4, where antibodies reactive against Ki67, a cell proliferation marker, reacted positively in the infracted hearts. This suggests that the cross-linked alginate solution of the invention is capable of inducing cell proliferation, or cell division, and, consequently, DNA synthesis. In other words, the cross-linked alginate solution triggers the surrounding tissue to re-enter the cell cycle.

Thus, one more aspect of the present disclosure is a method of inducing cardiac cell proliferation, comprising contacting said cells, *in vivo* or *in vitro*, with the cross-linked alginate solution or the composition of the invention.

Lastly, the present disclosure provides a kit for repairing damaged tissue, comprising:

(a) A polymer solution as defined in the invention, or a composition thereof;
(b) means for administering the polymer solution into the cardiac site of a patient in need;
(c) manual of instructions of how to use said polymer solution.

In one embodiment of the kit provided by the disclosure, said polymer is preferably a cross-linked alginate.

In another embodiment of the kit provided by the disclosure, said means for administering the polymer may be any one of a syringe with a 18-27G needle, any suitable percutaneous cardiac delivery system which includes a cardiac delivery device with a guidewire, including electromechanical mapping or MRI guided catheters, and any percutaneous cardiac device designed to assess the myocardium via the left ventricular cavity, the arterial or venous coronary system.

An important advantage of this concept of *in situ* tissue engineering, as presented herein, is the feasibility to introduce the implant, or the precursor material which will form it (like the cross-linked alginate solution of the present invention), with a catheter-based approach, thus avoiding the need for surgical thoracotomy. Therefore, the present work suggests that alginate injection is a new option that may create the milieu for stem cell homing, colonization and self repair.

The present invention is defined by the claims, the contents of which are to be read as included within the disclosure of the specification.

Disclosed and described, it is to be understood that this invention is not limited to the particular examples, process steps, and materials disclosed herein as such process steps and materials may vary somewhat.

It is also to be understood that the terminology used herein is used for the purpose of describing particular embodiments only and not intended to be limiting since the scope of the present invention will be limited only by the appended claims and equivalents thereof. It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following Examples are representative of techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

**Examples**

*Experimental Procedures*

The present study was performed in accordance with the guidelines of The Animal Care and Use Committee of Ben-Gurion University and Sheba Medical Center, Tel-Aviv University, which conforms to the policies of the American Heart Association and the "Guide for the Care and Use of Laboratory Animals" (Department of Health and Human Services, NIH Publication No. 85-23).

Preparation of the cross-linked alginate biomaterial

Sodium alginate (Mw ranging between 3 - 300 kDa, FMC Biopolymers, Drammen, Norway) was dissolved in double distilled water (DDW), to a final concentration of 2% (w/v). The alginate solution is cross-linked with calcium ions by adding 2% (w/v) calcium gluconate solution (D-gluconic acid, hemi-calcium salt, Sigma), while stirring intensively until a smoother solution was obtained. The viscosity of the cross-linked alginate solution can be manipulated by changing the weight ratio of calcium ions and alginate, as well as by judiciously selecting polymer molecular weight and composition (M/G ratio), nonetheless, in all cases the cross-linked alginates solution was of a viscosity that allowed it to be injected. Cross-linking was performed by a homogenizer.

A typical injection solution of cross-linked alginate is composed of 1% (w/v) alginate and 0.3% (w/v) of calcium gluconate and is prepared, for example, by mixing 1 ml of 2% (w/v) alginate, 0.3 ml of 2% (w/v) calcium gluconate solution and 0.7 ml of water, to yield a 2 ml cross-linked alginate composition. The cross-linked alginate is then placed at 4°C until use. In pigs, between 1-5 ml of the cross-linked alginate of the invention are injected. Between 0.2 to 5 ml of the cross-linked alginate may be injected into humans, although the optimal amount and frequency of injection shall be determined by the professional in charge of the patient under treatment.

The process developed by the inventors differs substantially from previous methods used to prepare alginate gels. In one method, alginate gels were prepared by a drop wise addition of sodium alginate solution to a concentrated solution of $CaCl_2$. This process involves a fast and extensive cross-linking of the alginate droplets, mainly on the surface of the drop. As the calcium ions diffuse into the drops, the alginate quickly changes from a viscous solution to a highly cross-linked solid gel. Such formulation is usually used for cell encapsulation and often involves another reaction between the alginate and positively-charged polymer to form a semi-permeable membrane [Lim, F. and Sun, A. M. (1980) Microencapsulated islets as bioartificial endocrine pancreas. Science 210:908-910]. In contrast, in the method of the present invention, the calcium ion solution is dispersed with the aqueous alginate solution using vigorous mixing, yielding a homogenous entanglement network of alginate biomaterial, which is held by calcium cross-linking.

In the method described in WO 94/25080, which is mostly applied for injectable alginate, calcium sulfate as solid particles is added to the alginate solution. Accordingly, alginate gelation rate depends on the solubility of calcium sulfate, which in turns depends on particle size of the salt. Usually, the salt particles have a wide range of size distribution which affects their solubility rate (the smaller the particle the faster it is dissolved). This results in an uncontrollable process of alginate gelation and the formation of non-homogenous gels. In contrast, the method developed by the present inventors enables the fabrication of a homogenous interpenetrating network, as shown by the rheology studies.

GPC-MALLS for determining molecular weight of alginate

Samples were separated on a chromatographic system comprising a Waters 606 pump followed by two PSS Suprema gel permeation columns connected in a series. Column description: dimensions 300 x 8 $mm^2$, particle size 10 mm, porosity of 3000 and 10,000 A. Flow rate was 0.5ml/min. The columns were kept at a constant temperature of 25°C inside a Techlab K-4 controlled oven. The chromatographic system was attached to a Dawn DSP (Wyatt Technology Corporation) multi-angle laser light scattering (MALLS) photometer equipped with a He/Ne laser working at 632.8 nm, a K5 refraction cell and 18 detectors at angles 14-163°. Concentration was monitored by a calibrated interferometric refractometer Optilab DSP (Wyatt Technology Corporation). Data processing and molar mass calculation were performed with Wyatt ASTRA software version 4.7. Each sample was injected three times to ensure reproducibility. The dn/dc of the alginate, measured with the Optilab DSP, controlled by Wyatt dn/dc software, was found to be 0.155 ml/g (aqueous buffer). Aqueous buffer solutions were prepared from ultra pure water (0.055 $\mu$s/cm, USF SERAL Purelab RO75 followed by USF SERAL Purelab UV) supplemented with 0.1 M $NaNO_3$, 0.02% (w/v) $NaN_3$ and 10 mM imidazole. The buffer was titrated with $NaNO_3$ to pH 7.0 and filtered through a 0.1 $\mu$m filter (Gelman Sciences VacuCap 60).

Rheological measurements

Rheological measurements were made on a CarriMed CLS50 controlled stress rheometer (CarriMed Instruments Ltd. Dorking, UK) operated in the cone-plate mode (cone angle 1° and 4° with diameters of 60 and 40 mm respectively). Small amplitude oscillatory shear experiments (0.1-10 Hz) were performed within the linear viscoelastic limit. Frequency scans were performed at the lowest stress possible to prevent damage to the sample. The linearity of the response was monitored continuously to ascertain linear viscoelasticity.

Rat Model of MI and Injection

The MI model was previously described by the inventors [Etzion *et al.* (2001) *id ibid.*; Leor *et* al. (1996) *id ibid.*]. Male Sprague-Dawley rats (~250 g) were anesthetized with a combination of 40 mg/kg ketamine and 10 mg/kg xylazine, intubated and mechanically ventilated. The chest was opened by left thoracotomy, the pericardium was removed and the proximal left coronary artery permanently occluded with an intramural stitch. One week after infarction, rats were anesthetized and under sterile technique the chest was opened. The infarcted area was identified visually on the basis of surface scar and wall motion akinesis. Rats were selected either to injection of 100-200 μL alginate based biomaterial or serum free culture medium using a 27-gauge needle. After injections into the scar, the surgical incision was sutured closed.

Histological and Immunohistochemical Examination

Eight weeks after injection, animals were sacrificed with an overdose of phenobarbital. Hearts were harvested, and processed for histological and immunohistochemical examination. Adjacent blocks were embedded in paraffin, sectioned into 5 μm slices and stained with hematoxylin and eosin. Serial sections were immunolabelled with antibodies against α-actin smooth muscle (SMA) isoform, which is present in embryonic cardiomyocytes but not in adult normal cardiomyocytes [Leor, J. et al. (1996) Circulation; 94:11332-336], fast myosin heavy chain (MHC) (Sigma), Ki67 (Novocastra Ltd.), SDF-1 (R&D systems).

Evaluation of Neovascularization

The effect of alginate injection upon neovascularization in the infarcted and peri-infarcted myocardium was assessed by immunohistologic staining of representative slides with anti-α-SMA antibodies (Sigma) to pericytes and arterioles. After low power examination, five consecutive adjacent fields were photographed from each section at a magnification of X200. The number of vessels was assessed from photomicrographs by computerized image analysis to count the number of vessels and to calculate vessel density (mean number of capillaries and arterioles/mm$^2$) in the hearts of transplanted and control groups.

Echocardiography to Evaluate Remodeling and Contractility

Transthoracic echocardiography was performed on all animals within 24 hours after MI (baseline echocardiogram) and at eight weeks after. Previous reports have demonstrated the accuracy and reproducibility of transthoracic echocardiography in rats [Etzion et al. (2001) *id ibid.;* Leor, J. *et al.* (2000) *id ibid.;* Litwin, S.E. et al. (1994) Circulation; 89:345-354; Schwarz, E.R. et al. (1998) Basic Res. Cardiol. 93:477-486]. Echocardiograms were performed with a commercially available echocardiography system equipped with 12 MHz phased-array transducer (Hewlett Packard) as previously reported. The parameters measured were: LV anterior wall thickness; maximal LV end-diastolic dimension; minimal left ventricular end-systolic dimension in M-mode and 2-D imaging; and fractional shortening as a measure of systolic function, which was calculated as FS (%)=[(LVIDd-LVIDs)/LVIDd]xI00, where LVID indicates LV internal dimension, s is systole, and d is diastole. Index of change in LV area (%) was calculated as [(EDA-ESA)/EDA]x100 where EDA indicates LV end diastolic area, ESA indicates LV end systolic area [Mehta, P.M. et al. (1988) J. Am. Coll. Cardiol. 11:630-636]. All measurements were averaged for three consecutive cardiac cycles and were performed by an experienced technician who was blinded to the treatment group.

Production of Myocardial Infarction in Swine

The methods used to create a myocardial infarction were the same as those described previously [Yau, T.M. et al. (2003) Ann Thorac Surg 75:169-176; Watanabe, E. et al. (1998) Cell Transplant 7:239-246]. In brief, female Sincklaire (mini) pigs were used, weighing 30-40 kg. All surgical procedures on swine were performed under general anesthesia and continuous electrocardiographic monitoring. Animals were pre-medicated with ketamine (20 to 30 mg/kg, intramuscular) before anesthetic induction with 4% isoflurane. Anesthesia was maintained with isoflurane 1% to 2.5%. The right femoral artery was isolated and cannulated with an introduction sheath. Through this, a cardiac catheter was placed in the mid portion of the left anterior descending artery (LAD) and an embolization coil (Boston Scientific, USA) was extruded from the catheter with a guide wire and placed in the distal portion of LAD under fluoroscopic guidance. This procedure induced a thrombus resulting in myocardial infarction in the left ventricle that was confirmed with angiography and electrocardiography. Electrical DC cardioversion was given when necessary.

Production of Mitral Regurgitation (MR)

Mitral regurgitation was produced by creating extensive posterior MI following coil embolization of the circumflex coronary artery.

Alginate-Biomaterial Injection

Alginate injection was performed at 7 to 10 days after MI. Pigs was anesthetized and under sterile technique the chest was opened. The infarcted area was identified visually by surface scar and wall motion abnormality. Pigs were randomized to two to three injections of alginate (up to 2.5 ml), or saline as control (up to 2.5 ml), into the infarcted myocardium. Air was expelled from the chest and the surgical incision sutured closed. In an initial series of pilot experiments, the technical aspects of the procedure were refined. Eight weeks after transplantation, the pigs were euthanized with phenobarbital overdose. The hearts was harvested, sectioned and processed for histology and immunohistochemistry.

Echocardiographic Evaluation of LV Remodeling and Function

Echocardiography was performed, under anesthesia, soon after MI, before transplantation, at 10 day after MI and at 30 and 60 days after, using a (2.5 MHz) phased-array transducer with an ultrasound system (Sonos 5500, Hewlett-Packard, Andover, Massachusetts). Images were recorded on VHS videotape. End-diastolic and end-systolic frames were selected from standard apical and parasternal views.

Global LV ejection fraction (LVEF) was estimated visually. LV volumes were measured by manually tracing the left ventricular cavity using the single plane modified Simpson's algorithm when 80% of the endocardial border could be detected in both the apical 4- and 2-chamber views, and by a single plane when 80% of the endocardial border could be detected only in the apical 4-chamber view. Regional myocardial assessment and wall motion score index were determined by assigning a segmental score (1 = normal, 2 = hypokinetic, 3 = akinetic, 4 = dyskinetic) to each of the 16 left ventricular segments, as recommended by the American Society of Echocardiography [Schiller, N.B. et al. (1989) J. Am. Soc. Echocardiogr. 2:358-367]. All segment scores were added and divided by the number of segments analyzed to obtain the wall motion score index.

The regional LV function was assessed by using the standard 16 segments [Schiller, N.B. et al. (1989) J. Am. Soc. Echocardiogr. 2:358-367]. For each segment, systolic wall motion and thickening were visually graded using the following semi-quantitative scoring system (1.4): 1 = normal or hyperkinesia; 2 = hypokinesia; 3 = akinesia; and 4 = dyskinesia. Left ventricular wall motion score index (WMSI) was derived using the sum of the individual scores divided by the total number of analyzed segments. Regional motion score index was calculated by the same method for the segments of the mid-LAD territory (infarct related artery territory). The studies were interpreted by a single experienced observer, and all measurements were obtained off line by a single technician.

MR was graded by color Doppler flow mapping using an algorithm that integrated jet expansion within the left atrium jet eccentricity, and size of the proximal area. MR was considered mild when regurgitant jet area occupied <20% of the LA area in the absence of a wall jet and a proximal isovelocity surface area visible without baseline shifting. It was considered severe in all patients in whom jet area was 40% of the LA area. Jet eccentricity or a sizable proximal flow convergence radius (0.6 mm in a patient with jet area <20%, and 0.9 mm in a patient with a jet area between 20% and 40%) raised the grade of MR by 1 degree.

Morphological and histological studies

After the invasive studies of ventricular function were completed, the heart was arrested with potassium-chloride and rapidly excised. The atria were removed and the heart was weighed. The coronary arteries were then perfused with 100 mL 10% formaldehyde, and the heart was fixed in diastole with an intraventricular pressure of 30 mm Hg in formaldehyde solution for 7 days before sectioning for histology. After fixation, the hearts was sliced into 5 mm thick slices and each section photographed. The mean scar length in each section was calculated as the mean of the epicardial scar length and the endocardial scar length. The scar area was then be calculated as the mean scar length for that section multiplied by 0.5 cm. Total scar area was calculated as the sum of scar areas for all sections. The thickness of the scar was calculated in each section, and scar volume was calculated as total scar area multiplied by the mean scar thickness. A cube of tissue from the center of the infarct zone measuring 5 mm on each side was embedded in paraffin and cut into 5 $\mu$m sections for staining with hematoxylin and eosin. For immunohistochemical studies, tissue slices were serially rehydrated in 100%, 95%, and 70% ethanol after deparaffinization with toluene. Endogenous peroxidase in the sample was blocked and the samples were stained with antibodies. Adjacent blocks were embedded in paraffin, sectioned into 5 $\mu$m slices and stained with hematoxylin and eosin. Serial sections were immunolabelled with antibodies against SMA, slow MHC (Sigma), Ki67 (Novocastra Ltd.) and SDF-1 (R&D systems).

Statistical Analysis

Univariate differences between the control and treated groups were assessed with t tests for continuous variables. Because each rat in both groups was used as her own control, changes between baseline and 8 weeks in the control and treated groups were assessed with paired t tests. Comparisons of the changes from baseline to 8 weeks in the control and treated groups were made with repeated-measures ANOVA using GraphPad Prism version 4.00 for Windows (GraphPad Software, San Diego, California, USA). The ANOVA model included the control versus treated and baseline versus 8 weeks as factors, and also included the interaction between the two factors [Perin, E.C. et al. (2003) Circulation; 107:2294-2302]. A probability value $p \leq 0.05$ was considered statistically significant.

Example 1

Preparation and Rheological Evaluation of Injectable Cross-Linked Alginate Biomaterial

The molecular weight (Mw) of the biopolymer alginate and its polydispersity (PD) (indication of the distribution range of molecular weight) may have an effect on the formation rate and structure of the resultant entanglement network. Thus, alginates Mw and PD using GPC-MALLS (as described in Experimental Procedures) were characterized (Table 1).

**Table 1: Alginate characterization**

| Alginate | Mn (g/mol) | Mw (g/mol) | PD (Mw/Mn) |
|---|---|---|---|
| LF 5/60 | 2.102e+4<br>2.113e+4 | 2.752e+4<br>2.656e+4 | 1.309±0.007<br>1.257±0.01 |
| LVG | 1.469e+5<br>1.385e+5 | 1.667e+5<br>1.559e+5 | 1.135±0.016<br>1.126±0.012 |
| MVG | 2.103e+5<br>2.055e+5 | 2.596e+5<br>2.385e+5 | 1.235+0.006<br>1.161±0.007 |
| Notes:<br>(i) The two numbers given for each measure represent two different batches of the material.<br>(ii) g/mol = Dalton | | | |

Rheology

The alginate gels fall under the category of physical gels, wherein physical cross-links are formed that are neither permanent nor as strong as covalent cross-links.

Steady shear viscosity

Viscosity $\eta$ is a measure of the resistance of a fluid to flow. It is defined as the ratio of shear stress $\tau$ to shear rate $\gamma$

$$(1) \qquad \eta = \tau / \gamma$$

When the fluid obeys equation (1) for all shear rates, it is denoted Newtonian [Ferry, J. D. (1980) Viscoelastic properties of polymers. John Wiley & Sons]. The sodium alginate solutions of 1 % (w/v) described herein are Newtonian (Table 2).

**Table 2: Viscosity as a function of shear rate (Shear rates $10^{-2}$-$10^{4}$(sec$^{-1}$))**

| Alginate Solution | Viscosity (cP) |
|---|---|
| 1% (w/v) LF 5/60 | 40 |
| 1% (w/v) LVG | 127 |
| 1% (w/v) MVG | 400 |

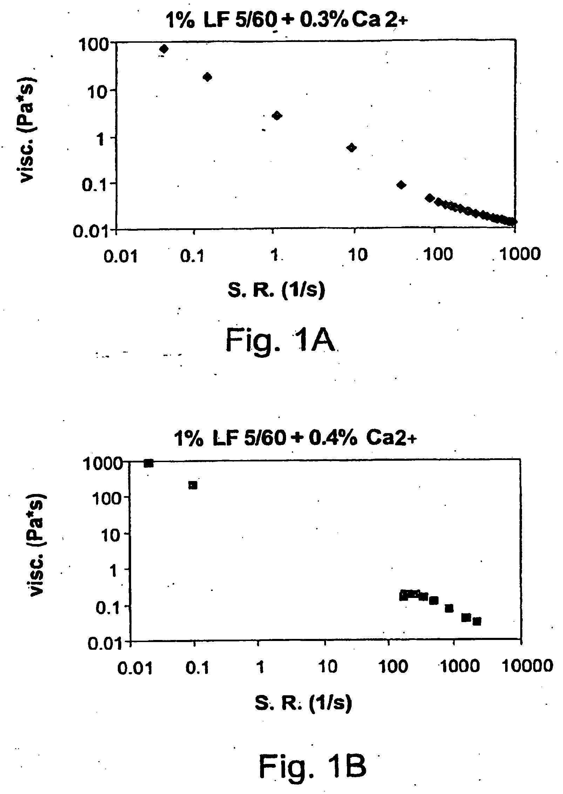

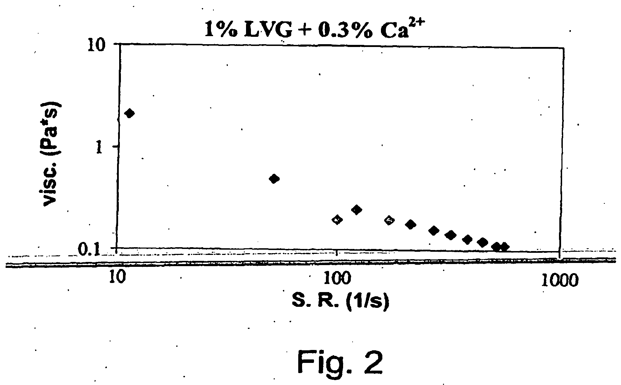

When calcium ions are added, they initiate cross-linking of the alginate, resulting in structuring and formation of entangled network. For calcium cross-linked alginate, the large deformation steady shear results are presented as plots of viscosity ($\eta$) versus shear rate (y). Figures 1 and 2 show an apparent viscosity, which varies with shear rate.

Such behavior is called shear thinning or pseudoplastic. A power-law relationship ($\eta \sim \gamma^{-1}$) was observed for a plot of log $\eta$ vs. log $\gamma$, which is typical of a structured material [Lapasin, R. and Pricl, S. (1995) Rheology of industrial polysaccharide: Theory and application. London, Blackbie, p. 620].

Small deformation oscillatory measurements

In order to obtain rheological information about a material (in the present case, the polymer-alginate solution), dynamic viscoelastic measurements are used. These are preferable to viscometric measurements, since the structure of the material is not disturbed, and information on both viscous and elastic properties of the material is obtained. The measurements are performed by applying a sinusoidal stress or strain of frequency f to the sample and measuring the response. The response is divided into (i) an elastic part in phase with the applied stress or strain, and (ii) a viscous part out of phase. Because of the two components, a complex notation is used. The complex shear modulus is denoted by G*, which is defined by the following formula:

$$G^* = G' + jG''$$

where G' is the storage modulus, i.e., the etastic part, G" is the loss modulus (the viscous part), and $j^2 = -1$.

A plot of the modulus as a function of frequency is often referred to as the mechanical spectrum of the material. The frequency dependence can be expressed by the slope n in a log-log plot of G' versus frequency, f, denoted by:

$$\text{Log } G' = n \log f + K$$

where K is a constant. In a physical gel, n>0, whereas in covalent gels n=0.

Small deformation oscillatory measurements are presented in terms of the storage modulus G' (the elastic response) and the loss modulus G" (viscous response) as functions of the angular frequency; G' being used as the primary indicator of a gel-like (structured) system. Figures 3 and 4 show the mechanical spectra of 1% (w/v) LF 5/60 alginate or LVG alginate samples, respectively, before and after the addition of different calcium ion concentration. For both the 1% (w/v) alginate solution (with no calcium ions), the value of G" exceeds that of G', which is a typical behavior of a random coil polysaccharide solution.

Upon the addition of calcium ions, the mechanical spectra of the resultant systems reveal G'-G" crossover, which is a typical feature for physical gels of the "entanglement network" type. This type of gel is distinguished from the strong covalent gels in that: (i) they do not have permanent cross links; (ii) they are strongly frequency-dependant; (iii) they have G'-G" crossover; and (iv) they flow as liquids at low frequencies. "Strong gels" have a permanent network (covalent) and show a shear modulus which is only slightly frequency-dependant [Clark, A. and Ross-Murphy, S.B. (1987) Structural and mechanical properties of biopolymer gels. Adv. Poly. Sci. Springer-Verlag, Berlin, Heidelberg]. Weak gels are intermediate type, and they are less frequency-dependant than the entanglement network and do not have G'-G" crossover at frequencies of $10^{-2}$-$10^2$ rad/s. They may have G'-G" crossover and behave differently at lower frequencies.

**Example 2**

**Comparing therapeutic effects of alginate biomaterial to cardiac cell transplantation in a rat model of MI**

Seven days after extensive MI, rats were randomized to alginate-based biomaterial injections, embryonic cardiomyocyte ($1.5 \times 10^6$) implantation, or medium injection into the myocardial scar. The alginate biomaterial was (calcium cross-linked, yet it still flowed under injection conditions, as described above. Echocardiography study was performed before and 1 and 2 months after implantation to assess LV remodeling and function. Hearts were harvested two months after implantation for histological evaluation.

Serial echocardiography studies revealed that the cross-linked alginate-based biomaterial injection enhanced scar thickness, prevented LV dilatation and dysfunction, comparable to cardiac cell transplantation, while control animals developed significant LV dilatation accompanied by progressive deterioration in LV contractility. The results are summarized in Table 3.

**Table 3**

| 2-D Echo | Alginate (n=7) | | Cells (n=5) | | Medium (n=4) | |
|---|---|---|---|---|---|---|
| | Before | 2m After | Before | 2 m After | Before | 2 m After |
| LVDD mm | 0.70±0.02 | 0.85±0.05 | 0.0.68±0.03 | 0.69±0.01 | 0.73±0.04 | 0.97±0.04* |
| LVSD mm | 0.52±0.05 | 0.65±0.06 | 0.45±0.02 | 0.45±0.04 | 0.56±0.04 | 0.80±0.04 |
| LVSA mm | 0.22±0.03 | 0.34±0.06 | 0.17±0.02 | 0.18±0.02 | 0.23±0.3 | 0.42±0.03* |
| LV FS% | 27±5 | 25±4 | 33±3 | 34±6 | 23±3 | 17±1* |

*2 months after vs. before; p<0.05
LVDD: LV diastolic dimension; LVSD: LV systolic dimension; LVSA: LV systolic area; FS: Fractional shortening.

These results suggest that injections of alginate-based biomaterial into the infracted myocardium in a rat model attenuate LV dilatation and myocardial dysfunction. These results are comparable to those achieved by embryonic cardiomyocytes transplantation. The results suggest a viable alternative to the difficulties in achieving appropriate cells to treat MI and CHF.

**Example 3**

**Animal model of MI treated with calcium cross-linked alginate biomaterial**

Overall, 39 rats were included in the study. Thirteen rats died after the surgical procedure to induce MI. Echocardiographic studies and analysis were performed on 24 rats. Fifteen rats were treated with alginate biomaterial injection and the control group (n=9) received injection of serum free culture medium. Two rats received injection of alginate into normal heart to study its safety and effect on normal myocardium.

- Echocardiography functional study

Alginate biomaterial injection significantly increased scar thickness (Table 4, p<0.0001). Furthermore, it efficiently attenuated the typical course of LV dilatation complicating extensive anterior MI (Figure 5, Table 4).
Although there was an increase in LV chamber internal diameters and areas, it was significantly less than that observed in control animals (Fig. 5 and Table 4). The beneficial effect of alginate biomaterial on LV remodeling was translated into prevention of LV dysfunction as reflected by attenuation in deterioration of fractional shortening and LV fractional area change (Fig. 6, Table 4).
Compared with biomaterial-treated rats, the control group displayed a typical course of extensive myocardial infarction, LV remodeling and heart failure. A significant increase in LV diastolic and systolic internal diameters was observed (Fig. 5 and 6, Table 4). LV end-diastolic and systolic cavity areas were markedly increased by 75% and more than 100% respectively (Figs. 5 and 6, Table 4; p<0.05). This process is similar to that observed in human patients after extensive anterior MI [Pilla, J.J. et al. (2002) Circulation 106:I207-211]. Progressive LV dilatation from baseline was also accompanied by significant deterioration in LV performance, reflected by the deterioration of fractional shortening (from 30±5% at baseline to 22±3%; p<0.05) and percentage of LV fractional area change (from 49±5% to 38±3%; p<0.05) at the end of the study (Fig. 7).

**Table 4: Results of echocardiography study**

| | Alginate biomaterial (n=15) | | | Control (n=9) | | |
|---|---|---|---|---|---|---|
| | Before | After | P | Before | After | p |
| M-mode | | | | | | |
| AW d cm | 0.14±0.01 | 0.15±0.01 | 0.11 | 0.14±0.01 | 0.14±0.02 | 0.6 |
| LVEDD cm | 0.71±0.02 | 0.86±0.03 | 0.004 | 0.74±0.02 | 0.98±0.03 | <0.0001 |
| LVESD cm | 50±0.03 | 65±0.04 | 0.01 | 0.51±0.04 | 0.78±0.05 | <0.0001 |
| *LV SF (%)* | 30±4 | 27±3 | 0.4 | 30±5 | 22±3 | 0.04 |
| 2-D | | | | | | |

(continued)

| | Alginate biomaterial (n=15) | | | Control (n=9) | | |
|---|---|---|---|---|---|---|
| | Before | After | P | Before | After | p |
| M-mode | | | | | | |
| AW d cm | 0.14±0.01 | 0.16±-0.01 | <0.001 | 0.14±0.01 | 0.14±0.01 | 0.8 |
| LVEDD cm | 0.71±0.02 | 0.86±0.03 | <0.01 | 0.73±0.02 | 0.98±0.03 | <0.0001 |
| LVESD cm | 0.50±0.03 | 0.65±0.04 | <0:01 | 0.51±0.04 | 0.78±0.05 | <0.0001 |
| LVED area cm$^2$ | 0.38±0.03- | 0.56±0.05 | 0.17 | 0.40±0.03 | 0.70±0.03 | <0.0001 |
| LVES area cm$^2$ | 0.21±0.03 | 0.32±0.04 | 0.05 | 0.20±0.02 | 0.44±0.04 | <0.0001 |
| FAC % | 47±4 | 45±3 | 0.6 | 49±4 | 38±3 | 0.02 |
| AW d- Anterior wall diastolic thickness<br>LVEDD- LV end diastolic dimension<br>LVESD - LV end systolic dimension<br>LV FS - LV fractional shortening - [(LVIDd-LVIDs)/LVIDd]x100<br>LV EDA- LV end diastolic area; LV ESA - LV end systolic area<br>FAC %- Fractional area change -[(EDA-ESA)/EDA]x100 | | | | | | |

Histological and Immunohistological Analysis

Injection of alginate biomaterial into normal myocardium resulted in intensive angiogenesis and migration of myofibroblasts as indicated by immunostaining with antibodies against α-SMA (Fig. 8A). Examination of sections of infarcted hearts treated with alginate injection showed intensive neovascularization and numerous myofibroblasts that populate the infarcted myocardium (Fig. 8B,C,D). Immunostaining with anti-Ki67 antibody (Fig. 11) of animals treated with injectable alginate biomaterial revealed positive staining in endothelial and cardiomyocytes at the infarct site, indicating DNA activity and replication. Thus, it is apparent that alginate induces cell replication which is associated with cardiac regeneration.

Microscopic examination of the slides immunostained with anti-SDF-1 antibody revealed strong expression of SDF-1 protein in endothelial cells, SMCs, fibroblast and, unexpectedly, in cardiomyocytes at the border zone (Fig. 9). Alginate biomaterial injections enhanced SDF-1 expression at infarcted site compared with non treated animals. Since SDF-1 is a key regulator of stem cell homing, this effect suggests that the injected biomaterial activates a signaling system that attracts stem cells to the injected site, which participate in neovascularization and myocardial regeneration.

Vessel density (mean number of capillaries and arterioles/mm$^2$±S.E.) in the infarcted myocardium of treated animals was significantly higher than control animals (231±13 vs. 180±16; p<0.02; Fig. 10).

**Example 4**

**Treatment of a Swine Model of Myocardial Infarction with Alginate injection**

Overall, 18 pigs were included in the study. Perioperative mortality was 27% (5 of 18). Echocardiographic studies and analysis were performed on 10 pigs with anterior MI. Five pigs were treated with alginate injection and control group (n=5) received injection of PBS. Four pigs were subjected to postero-lateral MI (MR.study).

- Immunohistochemistry

Eight weeks after LAD or circumflex (MR study) occlusion, the pig receiving alginate injection demonstrated high frequency of endothelial cells (Fig. 11A) and cardiomyocytes (Fig. 11B) with DNA activity, as determined by immunostaining with monoclonal antibodies reactive against Ki67. In contrast, in animals receiving saline there was a high frequency of cells with fibroblast morphology and reactivity with Ki67 within the infarct zone only. These results suggest that alginate biomaterial has a capability of inducing cell proliferation in cells such as cardiomyocytes, which normally do not have this activity in the adult heart. Such phenomenon is usually associated with cardiac regeneration.

### Example 5

**Echocardiography Functional Study: Reverse Ventricular Remodeling Reduces Ischemic Mitral Regurgitation (MR)**

Ischemic MR is a common complication of coronary artery disease that doubles late mortality [Lamas, G.A. et al. (1997) Circulation 96:827-833]. Extensive evidence has shown that ischemic MR results from left ventricular distortion, which displaces the papillary muscles (PMs) and tethers the mitral leaflets apically, restricting their closure. Therapy for ischemic MR, however, remains problematic. Mitral ring annuloplasty, often applied at the time of bypass surgery, reduces mitral annular size but does not directly address the broader problem of ischemic LV distortion with tethering; its benefits are therefore incomplete, particularly when LV remodeling continues to progress post-operatively. Uncertain benefit and the need for atrial incision and cardiopulmonary bypass can deter surgical repair. Repositioning the PMs using an external device may reduce ischemic MR.

The preliminary experiments presented herein show that injection of the cross-linked alginate based biomaterial repositions the PMs and reduces ischemic MR, without compromising LV function. Moreover, this relatively simple technique can be applied in the beating heart (Fig. 12).

### Example 6

**Injection of skeletal myoblast suspension in cross-linked alginate solution into the normal heart of rat**

Alginate biomaterial was injected into the heart muscle in order to promote angiogenesis and improve cell transplant retention and survival. This example shows that alginate biomaterial injections may be advantageous for the prolonged retention of co-injected cells, such as skeletal myoblasts.

Myoblasts from the hind limb muscle of Sprague-Dawley neonatal rats were isolated and purified according to the previously described procedure [Rosenblatt, J.D. (1995) In Vitro Cell Dev. Biol. Anim. 31:773-779]-. In order to verify the percentage of myoblasts in the population, cultured cells were stained with desmin (Sigma), which stains myoblasts (Fig. 13A). Injectable alginate solution was prepared as described herein above.

Male Sprague-Dawley rats ($\sim$250 g) were anesthetized with a combination of 40 mg/kg ketamine and 10 mg/kg xylazine, intubated and mechanically ventilated. The chest was opened by left thoracotomy, the pericardium removed and rats were subjected to injection of skeletal myoblasts, suspended in 100-200 $\mu$L alginate based biomaterial, using a 27-gauge needle, into the left ventricular free wall muscle. After injections into the heart muscle, the surgical incision was sutured closed.

Four weeks after injection, animals were sacrificed with an overdose of phenobarbital. Hearts were harvested, and processed for histological and immunohistochemical examination. Adjacent blocks were embedded in paraffin, sectioned into 5 $\mu$m slices and stained with hematoxylin and eosin. Serial sections were immunolabelled with antibodies against fast MHC (Sigma, UK).

As shown in Figure 13A-C, injection of myoblasts together with the cross-linked alginate solution of the invention induced neovascularization, as shown by the formation of functional new vessels, which can be evidenced by the presence of red blood cells (Fig. 13B). Furthermore, the co-injection with alginate biomaterial increased the retention of the transplanted myoblasts at injection site. The myoblasts differentiated into multinucleated fibers revealing skeletal striation.

### Example 7

**Injection of cross-linked alginate biomaterial enhances homing of stem cells to infarcted myocardium**

Athymic nude rats were subjected to myocardial infarction followed by injection of cross-linked alginate biomaterial into the infarcted tissue, At one week after infarction, animals were treated with intravenous infusion of human umbilical cord blood-derived CD133+ progenitor cells (2-4x10$^6$ cells). One week after transfusion, the hearts were harvested and representative sections were either fixed or frozen sectioned. The presence of human donor cells in the recipient heart was confirmed by Immunostaining for HLA-DR. HLA Immunostaining (Fig. 14, brown color) revealed that the infused donor cells homed and colonized the site of cross-linked alginate injection at the scar tissue. This experiment clearly demonstrates that cross-linked alginate injection into damaged tissue enhanced stem cell homing, which is consistent with the robust expression of SDF-1 at the site of injection.

Examples 6 and 7 show the advantageous effect of cross-linked alginate biomaterial on the retention of cells transplanted in beating hearts. In contrast, injection of cells in the absence of polymer, results in extensive cell leakage, and thus, most of the cells are not retained at the injection site, while at the same time, there is a high proportion of cell death.

**Claims**

1. An injectable aqueous cross-linked alginate solution for use in the treatment of cardiac arrhythmias, wherein the solution is cross-linked by bivalent cations and has an elastic response which becomes equal to or greater than its viscous response when small deformation oscillatory frequencies are applied and reveals shear thinning behaviour in a power-law relationship.

2. The solution according to the use as defined in claim 1, comprising 0.1-4% cross-linked alginate.

3. The solution according to the use as defined in claim 1 or 2, comprising 25 to 250 kDa alginate at a concentration of 0.5 to 2%.

4. The solution according to the use as defined in any of claims 1-3, comprising about 1% (w/v) alginate having a molecular weight of 3-300 kDa, and about 0.3% (w/v) calcium gluconate.

5. The solution according to the use as defined in any of the foregoing claims which is storage-stable for at least 24 hours.

6. The solution according to the use as defined in claim 5 which is storage-stable for more than one year.

7. The solution according to the use as defined in any of the foregoing claims, wherein the aqueous cross-linked alginate solution forms a gel in vivo when to be administered to a subject.

8. The solution according to the use as defined in any of claims 1-7 for treatment of focal arrhythmias.

9. The solution according to the use as defined in any of claims 1-7 for treatment of re-entrant arrhythmias.

10. The solution according to the use as defined in any of the foregoing claims 1 which is to be delivered to an ablation site.

11. The solution according to the use as defined in any of the foregoing claims which is to be delivered by injection into a re-entry pathway or an arrhythmia focus.

12. The solution according to the use as defined in any of the foregoing claims which is injected in a volume of 0.2 to 5ml.

13. Use of an injectable aqueous cross-linked alginate solution for the manufacture of a medicament for the treatment of cardiac arrhythmias, wherein the solution is cross-linked by bivalent cations and has an elastic response which becomes equal to or greater than its viscous response when small deformation oscillatory frequencies are applied and reveals shear thinning behaviour in a power-law relationship.

14. The use of claim 13, comprising 0.1-4% cross-linked alginate.

15. The use of claim 13 or 14, comprising 25 to 250 kDa alginate at a concentration of 0.5 to 2%.

16. The use of any one of claims 13-15, comprising about 1% (w/v) alginate having a molecular weight of 3-300 kDa, and about 0.3% (w/v) calcium gluconate.

17. The use of any one of claims 13-16, wherein the aqueous cross-linked solution forms a gel in vivo when to be administered to a subject.

18. The use of any of claims 13-17 for treatment of focal arrhythmias.

19. The use of any of claims 13-18 for treatment of re-entrant arrhythmias.

20. The use of any of any one of claims 13-19, wherein the aqueous cross-linked alginate solution is injected in a volume of 0.2 to 5ml.

**Patentansprüche**

1. Injizierbare wässrige quervernetzte Alginatlösung zur Verwendung in der Behandlung von Herzarrhythmien, wobei die Lösung durch zweiwertige Kationen quervernetzt ist und eine elastische Reaktion zeigt, die gleich oder größer wird als ihre viskose Reaktion, wenn oszillatorische Frequenzen kleiner Deformation angewendet werden, und die ein Scherverdünnungsverhalten in einer Potenzgesetzbeziehung zeigt.

2. Lösung gemäß der im Anspruch 1 definierten Verwendung, die 0,1-4% quervernetztes Alginat umfasst.

3. Lösung gemäß der im Anspruch 1 oder 2 definierten Verwendung, die 25 kDabis 250 kDa-Alginat in einer Konzentration von 0,5 bis 2% umfasst.

4. Lösung gemäß der in einem der Ansprüche 1 bis 3 definierten Verwendung, die etwa 1% (Gew./Vol.) Alginat mit einem Molekulargewicht von 3-300 kDA und etwa 0,3% (Gew./Vol.) Kalziumglukonat umfasst.

5. Lösung gemäß der in einem der vorangegangenen Ansprüche definierten Verwendung, die für mindestens 24 Stunden lagerstabil ist.

6. Lösung gemäß der im Anspruch 5 definierten Verwendung, die für mehr als ein Jahr lagerstabil ist.

7. Lösung gemäß der in einem der vorangegangenen Ansprüche definierten Verwendung, wobei die wässrige quervernetzte Alginatlösung in vivo ein Gel bildet, wenn sie an ein Individuum verabreicht werden soll.

8. Lösung gemäß der in einem der Ansprüche 1 bis 7 definierten Verwendung zur Behandlung von fokalen Arrhythmien.

9. Lösung gemäß der in einem der Ansprüche 1 bis 7 definierten Verwendung zur Behandlung von Reentry-Arrhythmien.

10. Lösung gemäß der in einem der vorangegangenen Ansprüche definierten Verwendung, die an eine Ablationsstelle verabreicht werden soll.

11. Lösung gemäß der in einem der vorangegangenen Ansprüche definierten Verwendung, die durch Injektion in die Reentry-Bahn oder einen Arrhythmieherd verabreicht werden soll.

12. Lösung gemäß der in einem der vorangegangenen Ansprüche definierten Verwendung, die in einem Volumen 0,2 bis 5 ml injiziert wird.

13. Verwendung einer injizierbaren wässrigen quervernetzten Alginatlösung für die Herstellung eines Medikaments zur Behandlung von Herzarrhythmien, wobei die Lösung durch zweiwertige Kationen quervernetzt ist und eine elastische Reaktion zeigt, die gleich oder größer wird als ihre viskose Reaktion, wenn oszillatorische Frequenzen kleiner Deformation angewendet werden, und die ein Scherverdünnungsverhalten in einer Potenzgesetzbeziehung zeigt.

14. Verwendung nach Anspruch 13, die 0,1-4% quervernetztes Alginat umfasst.

15. Verwendung nach Anspruch 13 oder 14, die 25 kDa- bis 250 kDa-Alginat in einer Konzentration von 0,5 bis 2% umfasst.

16. Verwendung nach einem der Ansprüche 13 bis 15, die etwa 1% (Gew./Vol.) Alginat mit einem Molekulargewicht von 3-300 kDA und etwa 0,3% (Gew./Vol.) Kalziumglukonat umfasst.

17. Verwendung nach einem der Ansprüche 13 bis 16, wobei die wässrige quervernetzte Lösung in vivo ein Gel bildet, wenn sie an ein Individuum verabreicht werden soll.

18. Verwendung nach einem der Ansprüche 13 bis 17 zur Behandlung von fokalen Arrhythmien.

19. Verwendung nach einem der Ansprüche 13 bis 18 zur Behandlung von Reentry-Arrhythmien.

20. Verwendung nach einem der Ansprüche 13 bis 19, wobei die wässrige quervernetzte Alginatlösung in einem Volumen von 0,2 bis 5 ml injiziert wird.

**Revendications**

1. Solution aqueuse injectable d'alginate réticulé pour une utilisation dans le traitement des arythmies cardiaques, dans laquelle la solution est réticulée par des cations bivalents et a une réponse élastique qui devient égale ou supérieure à sa réponse visqueuse quand de petites fréquences oscillatoires de déformation sont appliquées et révèle un comportement de fluidification par cisaillement selon une relation de loi de puissance.

2. Solution selon l'utilisation définie dans la revendication 1, comprenant de 0,1 à 4 % d'alginate réticulé.

3. Solution selon l'utilisation définie dans la revendication 1 ou 2, comprenant un alginate de 25 à 250 kDa à une concentration de 0,5 à 2 %.

4. Solution selon l'utilisation définie dans l'une quelconque des revendications 1 à 3, comprenant environ 1 % (p/v) d'alginate ayant un poids moléculaire de 3 à 300 kDa, et environ 0,3 % (p/v) de gluconate de calcium.

5. Solution selon l'utilisation définie dans l'une quelconque des revendications précédentes qui est stable au stockage pendant au moins 24 heures.

6. Solution selon l'utilisation définie dans la revendication 5 qui est stable au stockage pendant plus d'un an.

7. Solution selon l'utilisation définie dans l'une quelconque des revendications précédentes, dans laquelle la solution aqueuse d'alginate réticulé forme un gel in vivo quand elle doit être administrée à un sujet.

8. Solution selon l'utilisation définie dans l'une quelconque des revendications 1 à 7 pour le traitement des arythmies focales.

9. Solution selon l'utilisation définie dans l'une quelconque des revendications 1 à 7 pour le traitement des arythmies réentrantes.

10. Solution selon l'utilisation définie dans l'une quelconque des revendications précédentes qui doit être administrée à un site d'ablation.

11. Solution selon l'utilisation définie dans l'une quelconque des revendications précédentes qui doit être administrée par injection dans une voie de réentrée ou un foyer d'arythmie.

12. Solution selon l'utilisation définie dans l'une quelconque des revendications précédentes qui est injectée en un volume de 0,2 à 5 ml.

13. Utilisation d'une solution aqueuse injectable d'alginate réticulé pour la fabrication d'un médicament pour le traitement des arythmies cardiaques, dans laquelle la solution est réticulée par des cations bivalents et a une réponse élastique qui devient égale ou supérieure à sa réponse visqueuse quand de petites fréquences oscillatoires de déformation sont appliquées et révèle un comportement de fluidification par cisaillement selon une relation de loi de puissance.

14. Utilisation selon la revendication 13, comprenant de 0,1 à 4 % d'alginate réticulé.

15. Utilisation selon la revendication 13 ou 14, comprenant un alginate de 25 à 250 kDa à une concentration de 0,5 à 2 %.

16. Utilisation selon l'une quelconque des revendications 13 à 15, comprenant environ 1 % (p/v) d'alginate ayant un poids moléculaire de 3 à 300 kDa, et environ 0,3 % (p/v) de gluconate de calcium.

17. Utilisation selon l'une quelconque des revendications 13 à 16, dans laquelle la solution aqueuse réticulée forme un gel in vivo quand elle doit être administrée à un sujet.

18. Utilisation selon l'une quelconque des revendications 13 à 17 pour le traitement des arythmies focales.

**19.** Utilisation selon l'une quelconque des revendications 13 à 18 pour le traitement des arythmies réentrantes.

**20.** Utilisation selon l'une quelconque des revendications 13 à 19, dans laquelle la solution aqueuse d'alginate réticulé est injectée en un volume de 0,2 à 5 ml.

1% LF 5/60 + 0.3% Ca 2+

Fig. 1A

1% LF 5/60 + 0.4% Ca2+

Fig. 1B

Fig. 2

Fig. 3A

Fig. 3B

Fig. 3C

1% LVG

Fig. 4A

1% LVG + 0.3% Ca$^{2+}$

Fig. 4B

Fig. 5A

Fig. 5B

10/

Fig. 5C

Fig. 5D

Fig. 5E

Fig. 5F

Fig. 5G

Fig. 5H

Fig. 6A

Fig. 6B

Fig. 6C

Fig. 6D

Fig. 6E

Fig. 6F

Fig. 7A

Fig. 7B

Fig. 7C

Fig. 7D

Fig. 8A

Fig. 8B

Fig. 8C

Fig. 8D

Cont.

Fig. 9A

Fig. 9B

Fig. 9C

Fig. 10

Fig. 11A          Fig. 11B

Fig. 12A          Fig. 12B

Fig. 12C

Fig. 13A

Fig. 13B

Fig. 13C

HLA-DR

# Fig. 14

## REFERENCES CITED IN THE DESCRIPTION

### Patent documents cited in the description

- WO 9425080 A
- US 6134334 A
- WO 9915211 A
- US 5709854 A
- US 6129761 A
- US 6171610 B
- WO 9744070 A
- US 5776445 A

### Non-patent literature cited in the description

- **SUTTON, M.G. ; SHARPE, N.** *Circulation,* 2000, vol. 101, 2981-2988
- **MANN, D.L.** *Circulation,* 1999, vol. 100, 999-1008
- **JUGDUTT, B.I.** *Circulation,* 2003, vol. 108, 1395-1403
- **KHAND, A.U. et al.** *Eur. Heart J.,* 2001, vol. 22, 153-164
- **JESSUP, M. ; BROZENA, S.** *N. Engl. J. Med.,* 2003, vol. 348, 2007-2018
- **REDFIELD, M.M.** *N. Engl. J. Med.,* 2002, vol. 347, 1442-1444
- **ETZION, S. et al.** *J Mol Cell Cardiol,* 2001, vol. 33, 1321-1330
- **LEOR, J. et al.** *Expert Opin. Biol. Ther.,* 2003, vol. 3, 1023-39
- **BELTRAMI, A.P. et al.** *Cell,* 2003, vol. 114, 763-776
- **LEOR, J. et al.** *Circulation,* 2000
- **KAREN L. CHRISTMAN.** *International Conference on Engineering Tissue Growth,* 17 March 2003
- Remington's Pharmaceutical Sciences. Mack Publishing Co, 1990, 1521-1712
- **ETZION, S. et al.** *Am J Cardiovasc Drugs,* 2001, vol. 1, 233-244
- **KELLEY, S:T. et al.** *Circulation,* 1999, vol. 99, 135-142
- **PILLA, J.J. et al.** *Circulation,* 2002, vol. 106, 1207-211
- **SAAVEDRA, W.F. et al.** *J Am Coll Cardiol,* 2002, vol. 39, 2069-2076
- **LEMBCKE, A. et al.** *Eur J Cardiothorac Surg,* 2004, vol. 25, 84-90
- **KOLLET, O. et al.** *Blood,* 2001, vol. 97, 3283-3291
- **LAPIDOT, T. ; PETIT, I.** *Exp Hematol,* 2002, vol. 30, 973-981
- **ASKARI, A.T. et al.** *Lancet,* 2003, vol. 362, 697-703
- **PILLARISETTI, K. ; GUPTA, S.K.** *Inflammation,* 2001, vol. 25, 293-300
- **SALCEDO, R. et al.** *Am J Pathol,* 1999, vol. 154, 1125-1135
- **TACHIBANA, K. et al.** *Nature,* 1998, vol. 393, 591-594
- **SALVUCCI, O. et al.** *Blood,* 2002, vol. 99, 2703-2711
- **BROXMEYER, H.E. et al.** *J. Leukoc. Biol.,* 2003, vol. 73, 630-638
- **YAMAGUCHI J-I et al.** *Circulation,* 2003, vol. 107, 1322-1328
- **KOCHER, A.A. et al.** *Nat Med,* 2001, vol. 7, 430-436
- **SALISBURY, P.F. et al.** *Circ Res,* 1960, vol. 8, 794-800
- **BRAUNWALD, E.** *Circulation,* 1989, vol. 79, 441-444
- **HALE, S.L. ; KLONER, R.A.** *Am Heart J,* 1988, vol. 116, 1508-1513
- **ABBATE, A. et al.** *J Cell Physiol,* 2002, vol. 193, 145-153
- **ABBATE, A. et al.** *Circulation,* 2002, vol. 106, 1051-1054
- Department of Health and Human Services. *Guide for the Care and Use of Laboratory Animals*
- **LIM, F. ; SUN, A. M.** Microencapsulated islets as bi-oartificial endocrine pancreas. *Science,* 1980, vol. 210, 908-910
- **LEOR, J. et al.** *Circulation,* 1996, vol. 94, 11332-336
- **LITWIN, S.E. et al.** *Circulation,* 1994, vol. 89, 345-354
- **SCHWARZ, E.R. et al.** *Basic Res. Cardiol.,* 1998, vol. 93, 477-486
- **MEHTA, P.M. et al.** *J. Am. Coll. Cardiol.,* 1988, vol. 11, 630-636
- **YAU, T.M. et al.** *Ann Thorac Surg,* 2003, vol. 75, 169-176
- **WATANABE, E. et al.** *Cell Transplant,* 1998, vol. 7, 239-246

- **SCHILLER, N.B. et al.** *J. Am. Soc. Echocardiogr.,* 1989, vol. 2, 358-367
- **PERIN, E.C. et al.** *Circulation,* 2003, vol. 107, 2294-2302
- **FERRY, J. D.** Viscoelastic properties of polymers. John Wiley & Sons, 1980
- **LAPASIN, R. ; PRICL, S.** *Rheology of industrial polysaccharide: Theory and application,* 1995, 620
- Structural and mechanical properties of biopolymer gels. **CLARK, A. ; ROSS-MURPHY, S.B.** Adv. Poly. Sci. Springer-Verlag, 1987
- **PILLA, J.J. et al.** *Circulation,* 2002, vol. 106, I207-211
- **LAMAS, G.A. et al.** *Circulation,* 1997, vol. 96, 827-833
- **ROSENBLATT, J.D.** *In Vitro Cell Dev. Biol. Anim.,* 1995, vol. 31, 773-779